# DEMANDE DE BREVET EUROPEEN

(11) **EP 1 580 184 A1**
(43) Date de publication de la demande: **28.09.2005**
(21) Numéro de dépôt: 05290352.3
(22) Date de dépôt: 17.02.2005
(51) Int. Cl.: C07C 215/16, C07C 217/08, C07C 215/14, A61K 7/13

(54) **Para-phénylènediamines secondaires N-alkylpolyhydroxylées, composition tinctoriale les comprenant, procédés et utilisations**

(30) Priorité: 27.02.2004 FR 0402020
(71) Demandeur: L'OREAL, 75008 Paris (FR)
(72) Inventeur: Sabelle, Stephane, 75005 Paris (FR); Blaise, Christian, 94160 Saint-Mande (FR); Breton, Philippe, 78590 Noisy le Roi (FR); Vandenbossche, Jean-Jacques, 40400 Legaar (FR)
(74) Mandataire: Casalonga, Axel

(57) **Abrégé**

La présente demande concerne de nouvelles para-phénylènediamines secondaires N-alkylpolyhydroxylées, un procédé permettant leur préparation, leur utilisation en coloration d'oxydation capillaire, une composition pour la teinture des fibres kératiniques, et en particulier les fibres kératiniques humaines telles que les cheveux, contenant, dans un milieu approprié pour la teinture, au moins une telle para-phénylènediamine secondaire, un procédé de teinture des fibres kératiniques, l'utilisation de cette composition en coloration capillaire, ainsi qu'un "kit" de teinture.

## Description

La présente demande concerne une nouvelle famille de para-phénylènediamines secondaires N-alkylpolyhydroxylées, leur préparation et leur utilisation en coloration d'oxydation capillaire.

Il est connu de teindre les fibres kératiniques et en particulier les cheveux humains avec des compositions tinctoriales contenant des précurseurs de colorant d'oxydation, appelés généralement bases d'oxydation, tels que des ortho ou para-phénylènediamines, des ortho ou para-aminophénols et des composés hétérocycliques. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les méta-diaminobenzènes aromatiques, les méta-aminophénols, les méta-diphénols et certains composés hétérocycliques tels que des composés indoliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (c'est-à-dire abîmée) entre sa pointe et sa racine.

De manière surprenante et avantageuse, la Demanderesse vient de découvrir qu'il est possible d'obtenir une nouvelle famille de para-phénylènediamines secondaires N-alkylpolyhydroxylées capable de conduire à des colorations aux nuances variées, puissantes, esthétiques, peu sélectives et résistant bien aux diverses agressions que peuvent subir les fibres. La présente demande porte également sur un procédé de préparation de ces para-phénylènediamines secondaires N-alkylpolyhydroxylées, ainsi que sur leur utilisation en coloration d'oxydation capillaire.

Un autre objet de l'invention concerne des compositions pour la teinture des fibres kératiniques, en particulier des fibres kératiniques humaines telles que les cheveux, comprenant au moins une para-phénylènediamine secondaire portant un groupe polyhydroxylé permettant d'obtenir des colorations présentant les avantages précédemment mentionnés. En outre, ces compositions présentent un bon profil toxicologique.

L'invention a aussi pour objet un procédé de teinture mettant en oeuvre cette composition, l'utilisation de la composition selon la présente invention pour la teinture des fibres kératiniques, en particulier les fibres kératiniques humaines telles que les cheveux et un dispositif à plusieurs compartiments ou "kit" de teinture.

La composition de la présente invention permet en particulier d'obtenir une coloration de fibres kératiniques très puissante, peu sélective et tenace, en particulier à la lumière, tout en évitant la dégradation de ces fibres.

D'autres caractéristiques, aspects, objets et avantages de la présente invention apparaîtront encore plus clairement à la lecture de la description et des exemples qui suivent.

Les nouvelles para-phénylènediamines secondaires selon la présente invention sont des composés de formule générale (I) : 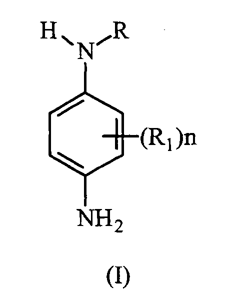 dans laquelle :
R représente un radical alkyle en C₂-C₁₈ linéaire ou ramifié, non substitué ou substitué par un ou plusieurs groupements amino, mono ou dialkyl(C₁-C₁₅)amino, alkyl(C₁-C₁₅)carbonyle, amido, mono ou dialkyl(C₁-C₁₅)amino carbonyle, ce radical alkyle pouvant être interrompu par un ou plusieurs hétéroatomes choisis parmi l'oxygène ou l'azote,
le radical R comprenant de 2 à 4 groupements hydroxyles ;
R₁ représente un atome d'hydrogène, un radical alkyle en C₁-C₁₅, un alcoxy en C₁-C₁₅, un hydroxy(alcoxy en (C₁-C₁₅)), un (alcoxy en (C₁-C₁₅))alkyle en C₁-C₁₅, un mono ou poly-hydroxy alkyle en C₁-C₁₅, ou un atome d'halogène;
n représente un entier variant de 1 à 4 ;
ou ses sels d'addition ;
à l'exception de la N(2,3-dihydroxypropyl) para-phénylènediamine.

De préférence, le groupement R de formule (I) représente un radical alkyle en C₂-C₁₈ linéaire ou ramifié comportant de 2 à 4 groupements hydroxyles ce radical alkyle pouvant être interrompu par un ou plusieurs hétéroatomes choisis parmi l'oxygène et l'azote ; et le groupement R₁ de formule (I) représente un atome d'hydrogène, un radical alkyle en C₁-C₆ ou un alcoxy en C₁-C₆.

De manière plus préférentielle, les composés de formule générale (I) sont choisis parmi :

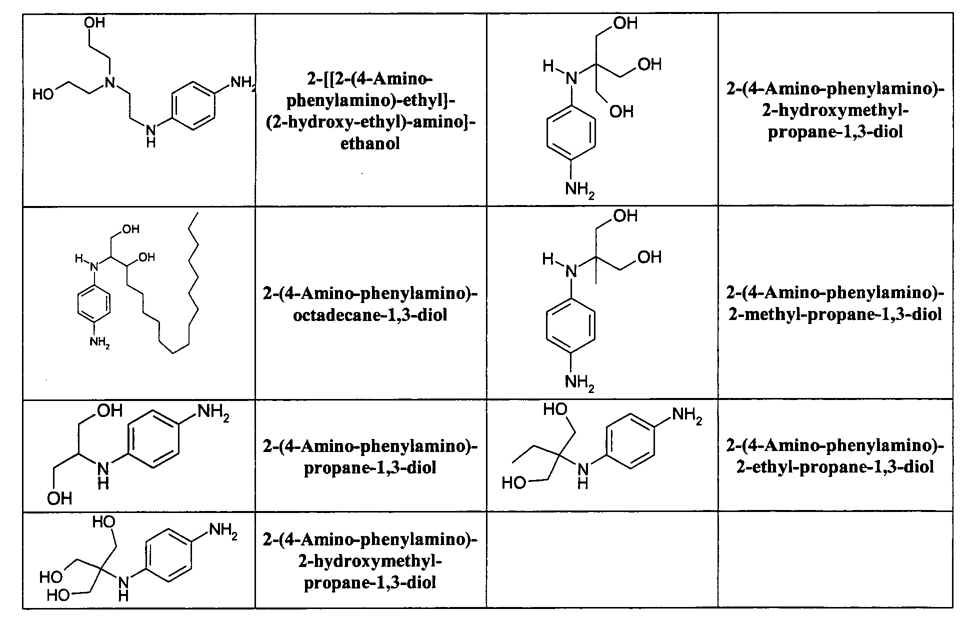

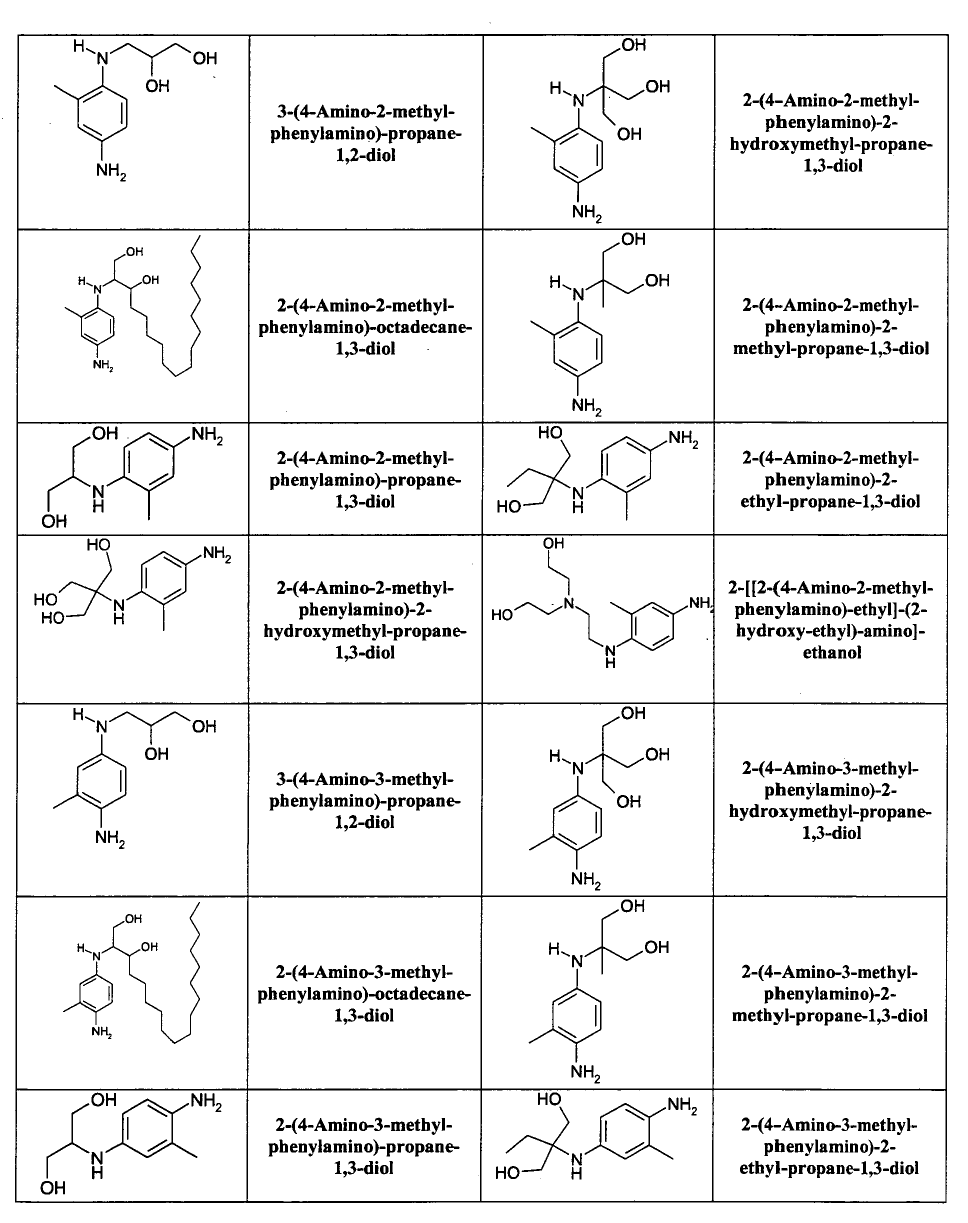

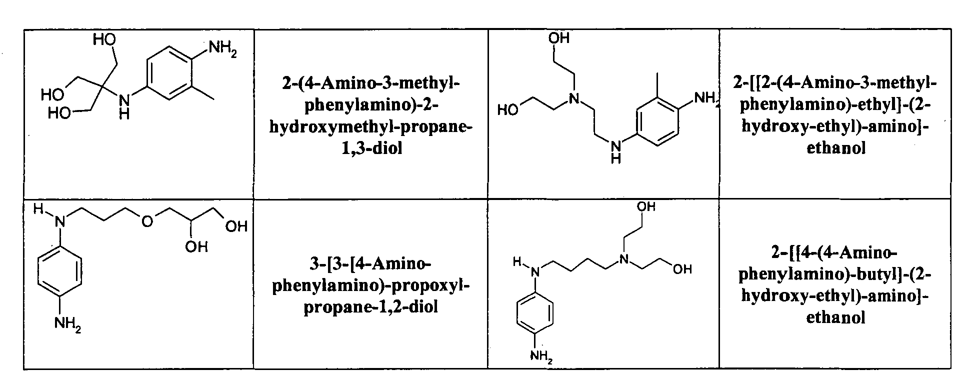

D'une manière générale, les sels d'addition utilisables pour les bases d'oxydation et les coupleurs sont notamment choisis parmi les sels d'addition avec un acide, tels que les chlorhydrates, les bromhydrates, les sulfates, les citrates, les succinates, les tartrates, les lactates, les tosylates, les benzènesulfonates, les phosphates et les acétates.

Les composés de formule (I) selon la présente demande peuvent être préparés de façon générale suivant une méthode qui comprend les étapes suivantes :
- substitution nucléophile de l'halogène en position para- du para-halogénonitrobenzène, par une amine primaire de formule RNH₂ en présence d'une base, R étant défini comme précédemment ;
- réduction de la fonction nitro du composé obtenu à l'étape précédente en fonction amine pour obtenir le composé de formule (I).

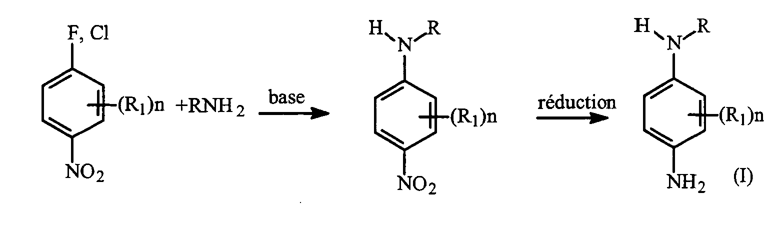

La première étape de synthèse est décrite dans les revues scientifiques Synthesis 1990 (12), 1147-1148 et Synth Commun 1990, 20(22), 3537-3545.

La deuxième étape est une étape de réduction classique, par exemple en effectuant une réaction d'hydrogénation par catalyse hétérogène en présence de Pd/C, Pd(II)/C, Ni de Raney ou encore en effectuant une réaction de réduction par un métal, par exemple par du zinc, fer, étain... (Advanced Organic Chemistry, 4th edition, 1992, J. MARCH, WILEY Interscience ; Reduction in Organic Chemistry, M. Hudlicky, 1983, Ellis Honwood series Chemical Science).

La présente demande concerne aussi les composés nitrés de formule (II) et les procédés de préparation des composés para-phénylènediamines secondaires de formule (I), dans lesquels on effectue une étape de réduction du composé nitré de formule (II) correspondant.

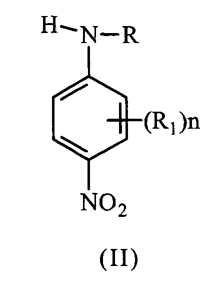

La présente demande porte également sur l'utilisation du composé de formule (I) suivante : 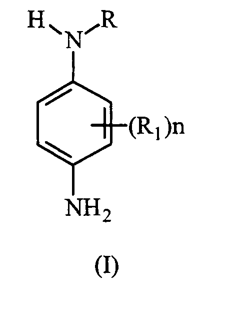 dans laquelle :
R représente un radical alkyle en C₂-C₁₈ linéaire ou ramifié, non substitué ou substitué par un ou plusieurs groupements amino, mono ou dialkyl(C₁-C₁₅)amino, alkyl(C₁-C₁₅)carbonyle, amido, mono ou dialkyl(C₁-C₁₅)amino carbonyle, ce radical alkyle pouvant être interrompu par un ou plusieurs hétéroatomes choisis parmi l'oxygène ou l'azote,
le radical R comprenant de 2 à 4 groupements hydroxyles ;R₁ représente un atome d'hydrogène, un radical alkyle en C₁-C₁₅, un alcoxy en C₁-C₁₅, un hydroxy(alcoxy en (C₁-C₁₅)), un (alcoxy en (C₁-C₁₅))alkyle en C₁-C₁₅, un mono ou poly-hydroxy alkyle en C₁-C₁₅, ou un atome d'halogène;
n représente un entier variant de 1 à 4 ;
ou ses sels d'addition,
à l'exception de la N(2,3-dihydroxypropyl) para-phénylènediamine.

La présente demande concerne aussi une composition cosmétique pour la teinture des fibres, de préférence les fibres kératiniques telles que les cheveux, comprenant, dans un milieu approprié pour la teinture, au moins un composé de formule générale (I) : 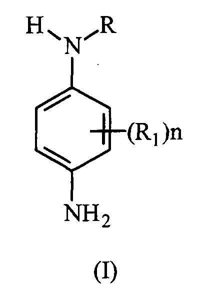 dans laquelle :
R représente un radical alkyle en C₂-C₁₈ linéaire ou ramifié, non substitué ou substitué par un ou plusieurs groupements amino, mono ou dialkyl(C₁-C₁₅)amino, alkyl(C₁-C₁₅)carbonyle, amido, mono ou dialkyl(C₁-C₁₅)amino carbonyle, ce radical alkyle pouvant être interrompu par un ou plusieurs hétéroatomes choisis parmi l'oxygène ou l'azote,
le radical R comprenant de 2 à 4 groupements hydroxyles ;
R₁ représente un atome d'hydrogène, un radical alkyle en C₁-C₁₅, un alcoxy en C₁-C₁₅, un hydroxy(alcoxy en (C₁-C₁₅)), un (alcoxy en (C₁-C₁₅))alkyle en C₁-C₁₅, un mono ou poly-hydroxy alkyle en C₁-C₁₅, ou un atome d'halogène;
n représente un entier variant de 1 à 4 ;
ou ses sels d'addition,
à l'exception de la N(2,3-dihydroxypropyl) para-phénylènediamine.

De préférence, le groupement R de formule (I) représente un radical alkyle en C₂-C₁₈ linéaire ou ramifié comportant de 2 à 4 groupements hydroxyles, ce radical alkyle pouvant être interrompu par un ou plusieurs hétéroatomes choisis parmi l'oxygène et l'azote ; et le groupement R₁ de formule (I) représente un atome d'hydrogène, un radical alkyle en C₁-C₆ ou un alcoxy en C₁-C₆.

De manière plus préférentielle, les composés de formule générale (I) sont choisis parmi :

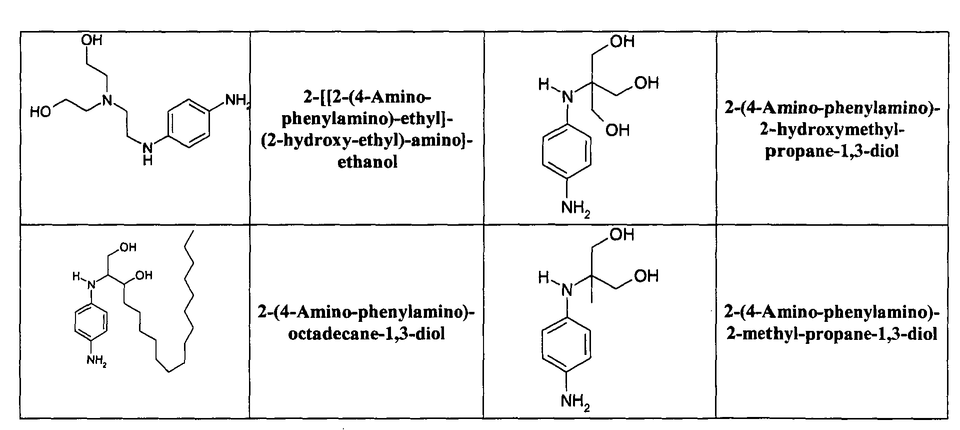

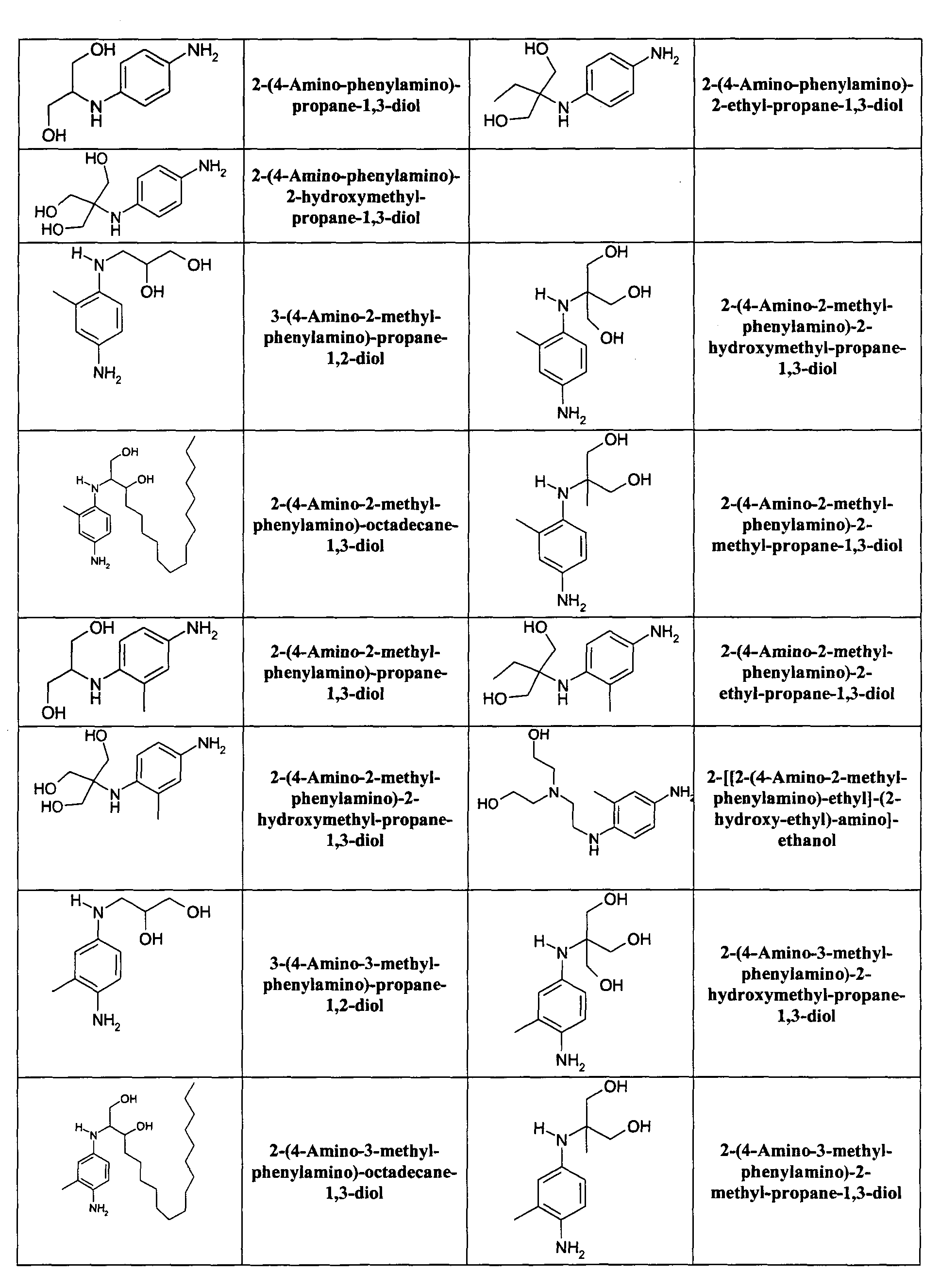

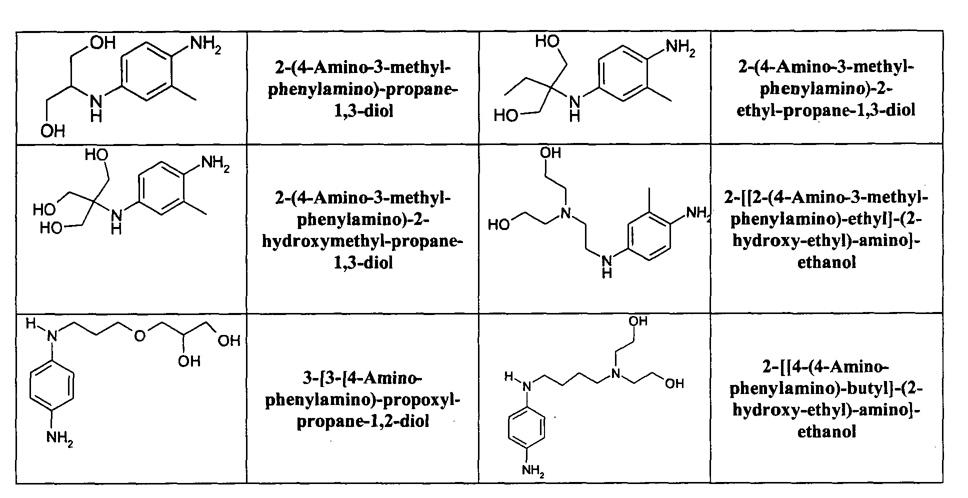

D'une manière générale, les sels d'addition utilisables pour les bases d'oxydation et les coupleurs sont notamment choisis parmi les sels d'addition avec un acide, tels que les chlorhydrates, les bromhydrates, les sulfates, les citrates, les succinates, les tartrates, les lactates, les tosylates, les benzènesulfonates, les phosphates et les acétates..

De préférence, la concentration du composé de formule générale (I) est comprise entre 0,0001 et 20%, de préférence entre 0,005 à 6% en poids par rapport au poids total de la composition.

Le milieu approprié pour la teinture est avantageusement constitué par de l'eau ou par un mélange d'eau et d'au moins un solvant organique tel que, par exemple, les alcools inférieurs en C₁-C₄, ramifiés ou non, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, le glycérol ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

Avantageusement, la composition cosmétique comprend au moins un adjuvant cosmétique choisi dans le groupe formé par les agents anti-oxydants, les agents de pénétration, les agents séquestrants, les parfums, les tampons, les agents dispersants, les tensioactifs, les agents conditionneurs, les agents filmogènes, les polymères, les céramides, les agents conservateurs, les agents nacrants ou opacifiants, les vitamines ou provitamines.

Les adjuvants ci-dessus sont en général présents en quantité comprise pour chacun d'eux entre 0,01 et 20 % en poids par rapport au poids de la composition.

La composition selon l'invention contient de préférence au moins un coupleur d'oxydation.

Parmi les coupleurs d'oxydation, on peut notamment citer les méta-phénylènediamines, les méta-aminophénols, les méta-diphénols, les coupleurs naphtaléniques et les coupleurs hétérocycliques ainsi que leurs sels d'addition.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène (ou résorcinol), le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine, la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition.

Généralement la concentration du ou des coupleurs d'oxydation est comprise entre 0,0001 et 20%, de préférence entre 0,005 à 6% en poids par rapport au poids total de la composition.

La composition selon l'invention peut également contenir des bases d'oxydation additionnelles différentes du composé de formule (I).

Les bases d'oxydation classiques peuvent notamment être choisies parmi les para-phénylènediamines, les bis-phénylalkylènediamines, les para-aminophénols, les ortho-aminophénols et les bases hétérocycliques et leurs sels d'addition.

Parmi les para-phénylènediamines, on peut plus particulièrement citer à titre d'exemple, la para-phénylènediamine, la para-toluènediamine, la 2-chloro para-phénylènediamine, la 2,3-diméthyl para-phénylènediamine, la 2,6-diméthyl para-phénylènediamine, la 2,6-diéthyl para-phénylènediamine, la 2,5-diméthyl para-phénylènediamine, la N,N-diméthyl para-phénylènediamine, la N,N-diéthyl para-phénylènediamine, la N,N-dipropyl para-phénylènediamine, la 4-amino N,N-diéthyl 3-méthyl aniline, la N,N-bis-(β-hydroxyéthyl) para-phénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl para-phénylènediamine, la 2-fluoro para-phénylènediamine, la 2-isopropyl para-phénylènediamine, la N-(β-hydroxypropyl) para-phénylènediamine, la 2-hydroxyméthyl para-phénylènediamine, la N,N-diméthyl 3-méthyl para-phénylènediamine, la N,N-(éthyl, β-hydroxyéthyl) para-phénylènediamine, la N-(β,γ-dihydroxypropyl) para-phénylènediamine, la N-(4'-aminophényl) para-phénylènediamine, la N-phényl para-phénylènediamine, la 2-β-hydroxyéthyloxy para-phénylènediamine, la 2-β-acétylaminoéthyloxy para-phénylènediamine, la N-(β-méthoxyéthyl) para-phénylènediamine, la 4 aminophenyl pyrrolidine, le 2 thiényl para-phénylène diamine, le 2-β hydroxyéthylamino 5-amino toluène, la 3-hydroxy 1-(4' aminophenyl)pyrrolidine, la 6-(4-Amino-phenylamino)-hexan-1-ol et leurs sels d'addition avec un acide.

Parmi les para-phénylènediamines citées ci-dessus, la para-phénylènediamine, la para-toluènediamine, la 2-isopropyl para-phénylènediamine, la 2-β-hydroxyéthyl para-phénylènediamine, la 2-β-hydroxyéthyloxy para-phénylènediamine, la 2,6-diméthyl para-phénylènediamine, la 2,6-diéthyl para-phénylènediamine, la 2,3-diméthyl para-phénylènediamine, la N,N-bis-(β-hydroxyéthyl) para-phénylènediamine, la 2-chloro para-phénylènediamine, la 2-β-acétylaminoéthyloxy para-phénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 2-méthyl phénol, le 4-amino 3-méthyl phénol, le 4-amino 3-fluoro phénol, le 4-amino 3-chloro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, le 4-amino-2,6-dichlorophénol, le 4-amino-6[((5'-amino-2'-hydroxy-3'-méthyl)phényl)méthyl]-2-méthylphénol, le bis(5'-amino-2'-hydroxy)phénylméthane et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 3,4-diamino pyridine, et leurs sels d'addition avec un acide.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs sels d'addition avec un acide.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine et leurs sels d'addition et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition.

Généralement la concentration de la ou des bases d'oxydation additionnelle est comprise entre 0,0001 et 20%, de préférence entre 0,005 à 6% en poids par rapport au poids total de la composition.

D'une manière générale, les sels d'addition utilisables pour les bases d'oxydation et les coupleurs sont notamment choisis parmi les sels d'addition avec un acide, tels que les chlorhydrates, les bromhydrates, les sulfates, les citrates, les succinates, les tartrates, les lactates, les tosylates, les benzènesulfonates, les phosphates et les acétates.

La composition tinctoriale conforme à l'invention peut en outre contenir un ou plusieurs colorants directs pouvant notamment être choisis parmi les colorants nitrés de la série benzénique, neutres, acides ou cationiques, les colorants directs azoïques neutres, acides ou cationiques, les colorants directs quinoniques et en particulier anthraquinoniques neutres, acides ou cationiques, les colorants directs aziniques, les colorants directs méthiniques, azométhiniques, triarylméthaniques, indoaminiques et les colorants directs naturels. De préférence, la composition selon l'invention comprend au moins un colorant choisi parmi les colorants directs cationiques et les colorants directs naturels.

Parmi les colorants directs cationiques utilisables selon l'invention, on peut citer les colorants directs azoïques cationiques décrits dans les demandes de brevets WO 95/15144, WO-95/01772 et EP-714954.

Parmi ces composés on peut tout particulièrement citer les colorants suivants :
- chlorure de 1,3-diméthyl-2-[[4-(diméthylamino)phényl]azo]-1H-Imidazolium,
- chlorure de 1,3-diméthyl-2-[(4-aminophényl)azo]-1H-Imidazolium,
- méthylsulfate de 1-méthyl-4-[(méthylphénylhydrazono) méthyl]-pyridinium.

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

Le ou les colorants directs représentent de préférence de 0,001 à 20% en poids environ du poids total de la composition prête à l'emploi et encore plus préférentiellement de 0,005 à 10% en poids environ.

Bien entendu, l'homme de l'art veillera à choisir le ou les adjuvants, précurseurs de colorants d'oxydations additionnels, coupleurs d'oxydation, colorants directs de manière telle que les propriétés avantageuses attachées intrinsèquement à la composition de teinture d'oxydation conforme à l'invention ne soient pas, ou substantiellement pas, altérées par la ou les adjonctions envisagées.

Une composition tinctoriale prête à l'emploi est obtenue par ajout un ou plusieurs agents oxydants classiquement utilisés pour la teinture d'oxydation des fibres kératiniques, tels que par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases, le peroxyde d'hydrogène étant particulièrement préféré, à la composition tinctoriale telle que précédemment décrite.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques autres que les diacides carboxyliques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule : 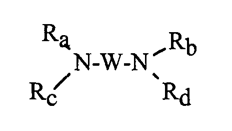 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de la présente demande concerne un procédé dans lequel on applique sur les fibres la composition telle que définie précédemment, et qu'on révèle la couleur à l'aide d'un agent oxydant. La couleur peut être révélée à pH acide, neutre ou alcalin. L'agent oxydant peut être ajouté à la composition de l'invention juste au moment de l'emploi. Il peut être mis en oeuvre à partir d'une composition oxydante le contenant, appliquée simultanément ou séquentiellement sur les fibres au moment de l'application de la composition tinctoriale.

Selon un mode de réalisation particulier, la composition selon la présente invention est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps de pose de 3 à 50 minutes environ, de préférence 5 à 30 minutes environ, les fibres kératiniques sont rincées, lavées au shampooing, rincées à nouveau puis séchées.

La composition oxydante peut renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques et tels que définis précédemment.

La composition prête à l'emploi qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

La présente demande concerne également l'utilisation de la composition cosmétique selon l'invention comprenant, dans un milieu approprié pour la teinture, au moins un composé de formule générale (I) pour la teinture des fibres, de préférence les fibres kératiniques telles que les cheveux.

L'invention a aussi pour objet un dispositif à plusieurs compartiments ou "kit" de teinture dans lequel un premier compartiment renferme la composition tinctoriale définie ci-dessus et un deuxième compartiment renferme une composition oxydante. Ce dispositif peut être équipé d'un moyen permettant de délivrer sur les cheveux le mélange souhaité, tel que les dispositifs décrits dans le brevet FR-2 586 913 au nom de la demanderesse.

A partir de ce dispositif, il est possible de teindre les fibres kératiniques à partir d'un procédé qui comprend le mélange d'une composition tinctoriale conforme à l'invention avec un agent oxydant tel que défini précédemment, et l'application du mélange obtenu sur les fibres kératiniques pendant un temps suffisant pour développer la coloration désirée.

Les exemples qui suivent servent à illustrer l'invention sans toutefois présenter un caractère limitatif.

### EXEMPLES

### -A- EXEMPLES DE SYNTHESE

### Exemple 1 : Synthèse du 2-(4-Amino-phénylamino)-2-méthyl-propane-1,3-diol, dichlorhydrate (2)

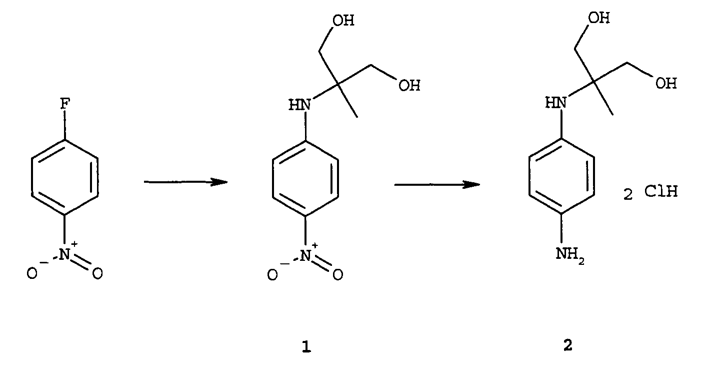

### Etape 1 : Préparation du 2-Méthyl-2-(4-nitro-phénylamino)-propane-1,3-diol (1)

On fond 43 g de 2-amino-2-méthyl,1-3 propanediol vers 120°C puis on ajoute 21,15g (0,15 mole) de 1-fluoro-4-nitrobenzène. Le mélange est chauffé 1 heure à 180°C.

Lorsque la réaction est totale, le milieu est versé sur un mélange glace/eau en agitant fortement. Le précipité jaune formé est filtré, lavé à l'eau puis séché pour donner 27,6g de solide.

Après recristallisation dans l'éthanol, l'analyse élémentaire donne les résultats suivants :

| | **THEORIE** | **TROUVE** |
|---|---|---|
| C | 53.09 | 53.13 |
| H | 6.24 | 6.28 |
| N | 12.38 | 12.34 |
| O | 28.29 | 28.37 |

### Etape 2 : Préparation du 2-(4-phénylamino)-méthyl-propane-1,3-diol, dichlorhydrate (2)

22,6 g (0,1 mole) de 2-méthyl-2-(4-nitro-phénylamino)-propane-1,3-diol (1) sont placés dans un mélange d'alcool, d'eau et de cyclohexène dans les proportions suivantes 67 ml/5ml/46ml. On ajoute 10g de catalyseur Pd/C 10% et on porte le mélange au reflux 2 heures. Le milieu est filtré à chaud ; le catalyseur est lavé avec l'alcool. Le filtrat est concentré à sec puis repris dans l'alcool. Après refroidissement, 50 ml d'éthanol chlorhydrique 7N ont été ajoutés. Le précipité formé est filtré, lavé à l'alcool puis à l'éther isopropylique et enfin à l'éther de pétrole. Après séchage, on obtient 23,4 g de produit attendu.

Après recristallisation, l'analyse élémentaire donne les résultats suivants :

| | **THEORIE** | **TROUVE** |
|---|---|---|
| C | 44.62 | 44.14 |
| H | 6.74 | 6.77 |
| N | 10.41 | 10.26 |
| O | 11.89 | 12.49 |
| Cl | 26.34 | 26.10 |

Les spectres de RMN du proton et de masse sont conformes à la structure attendue du produit.

### Exemple 2 : Synthèse de 2-(4-Amino-phénylamino)-octadécane-1,3-diol, dichlorhydrate (4)

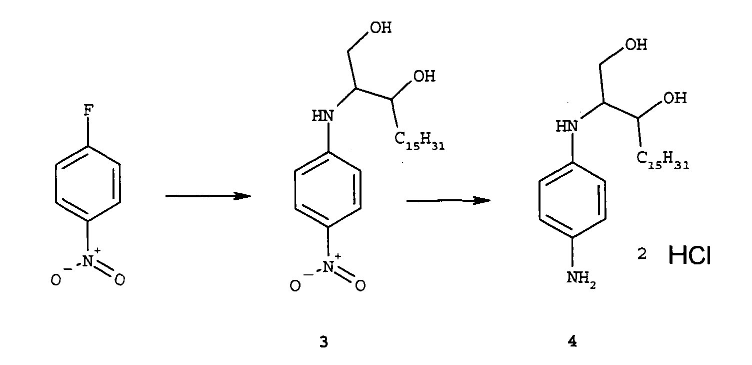

### Etape 1 : préparation de 2-(4-Nitro-phénylamino)-octadécane-1,3-diol (3)

Dans un réacteur de 250 ml équipé d'un réfrigérant, introduire 13 g (0,092 mole) de 4-fluoronitrobenzène, 30g (0,099 mole) de 2-Amino-octadécane-1,3-diol et 11 g (0,104 mole) de carbonate de sodium. Porter le mélange réactionnel à 120°C pendant 3 heures. Ajouter lentement 200ml d'eau au milieu réactionnel puis le verser sur 500 ml d'eau glacée. Filtrer sur verre fritté le précipité formé et le sécher sous vide à 50°C en présence P₂O₅. Après deux recristallisations dans l'éthanol, on isole 10 g de poudre jaune.

| | **THEORIE** | **TROUVE** |
|---|---|---|
| C | 68.21 | 68.26 |
| H | 10.02 | 10.16 |
| N | 6.63 | 6.46 |
| O | 15.14 | 14.47 |

### Etape 2 : Préparation de 2-(4-Amino-phénylamino)-octadécane-1,3-diol, dichlorhydrate (4)

Dans un réacteur de 250 ml équipé d'un réfrigérant, mettre en suspension 10 g (0,0237 mol) de 2-(4-Nitro-phénylamino)-octadécane-1,3-diol (3) dans 70 ml d'éthanol. Ajouter 6 g de Pd/C à 10% humide à 50% et 60 ml de cyclohexène. Porter le mélange réactionnel au reflux pendant 2h. Filtrer à chaud sur célite puis concentrer de moitié. Refroidir le milieu à 5°C par un bain de glace puis faire buller lentement HCl gazeux , le produit précipite. Filtrer sur verre fritté puis laver avec un peu d'éther isopropylique. Sécher sous vide en présence de P₂O₅ à 50°C. On obtient 9,6 g d'une poudre blanche.

| | **THEORIE** | **TROUVE** |
|---|---|---|
| C | 61.92 | 61.85 |
| H | 9.96 | 10.05 |
| N | 6.02 | 5.93 |
| O | 6.87 | 6.86 |
| Cl | 15.23 | 15.13 |

Les spectres de RMN du proton et de masse sont conformes à la structure attendue du produit.

### Exemple 3 :Synthèse de 2-(4-Amino-phénylamino)-2-hydroxyméthyl-propane-1,3-diol(6)

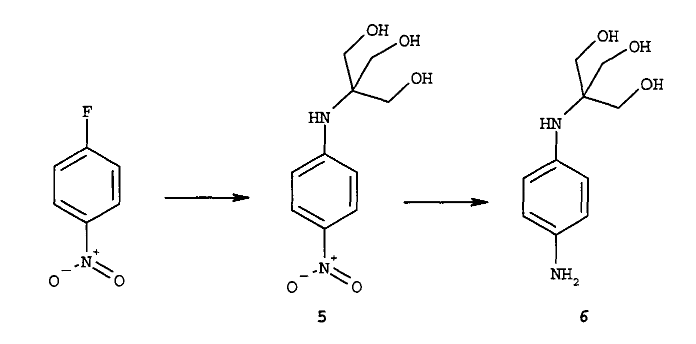

### Etape 1 : Préparation de 2-Hydroxyméthyl-2-(4-Nitro-phénylamino)-propane-1,3-diol (5)

On fond 43 g de tris-hydroxyméthylaminométhane vers 165°C puis on ajoute 21,15 g (0,15 mole) de 1-fluoro-4-nitrobenzène vers 170-180°C. Le mélange est laissé agité à 170°C pendant 30 min puis chauffé 1 heure à 190°C. Lorsque la réaction est totale, le milieu est versé sur un mélange glace/eau en agitant fortement. Le précipité jaune formé est filtré, lavé à l'eau et 3 fois à l'alcool. Après recristallisation dans l'éthanol, on isole 14 g de produit attendu.

L'analyse élémentaire donne les résultats suivants :

| | **THEORIE** | **TROUVE** |
|---|---|---|
| C | 49.59 | 49.78 |
| H | 5.83 | 5.80 |
| N | 11.56 | 11.20 |
| O | 33.02 | 32.82 |

### Etape 2 : Préparation du 2-(4-Amino-phénylamino)-2-hydroxyméthyl-propane-1,3-diol (6)

7,3 g (0,03 mole) de 2-Hydroxyméthyl-2-(4-Nitro-phénylamino)-propane-1,3-diol (5) sont placés dans un mélange d'alcool, d'eau et de cyclohexène dans les proportions suivantes 30ml/15ml/3ml. On ajoute 3g de catalyseur Pd/C 10% et on porte le mélange au reflux 2 heures jusqu'à décoloration. Le milieu est filtré à chaud ; le catalyseur est lavé avec l'alcool. Le filtrat est concentré à sec puis repris dans 10ml d'éthanol, le précipité formé est filtré, lavé à l'alcool puis à l'isopropyle éther. Après séchage, on obtient 3,7 g d'une poudre blanche dont l'analyse élémentaire donne les résultats suivants :

| | **THEORIE** | **TROUVE** |
|---|---|---|
| C | 56.59 | 56.54 |
| H | 7.60 | 7.60 |
| N | 13.20 | 13.20 |
| O | 22.61 | 22.81 |

Les spectres de RMN du proton et de masse sont conformes à la structure attendue du produit.

### Exemple 4 : Synthèse du 3-(4-Amino-2-methyl-phenylamino)-propane-1,2-diol, dichlorhydrate (8)

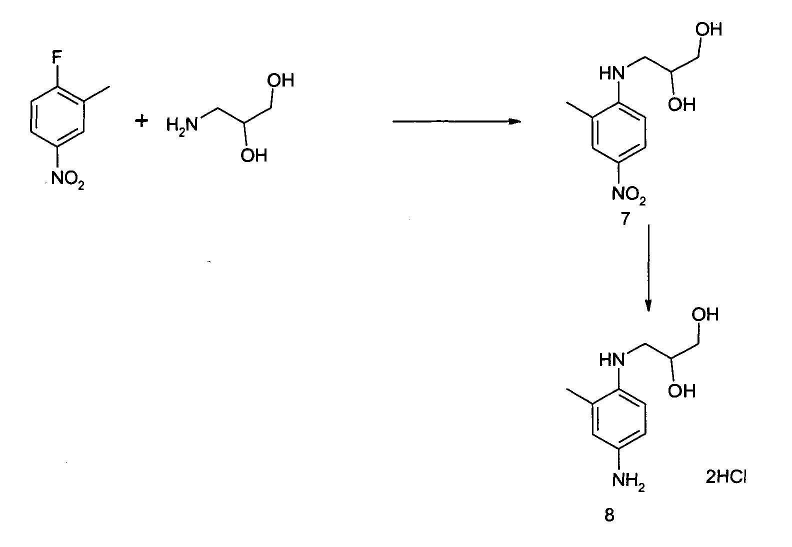

### Etape 1 : synthèse du 3-(4-nitro-2-methyl-phenylamino)-propane-1,2-diol (7)

A une solution de 20 ml de N-méthyl-pyrrolidinone, 1,4 g de 2,3-dihydroxy-1-propylamine et 2,14 g de K₂CO₃ , on ajoute 2 g de 2-fluoro-5-nitrotoluène. Le milieu réactionnel est chauffé à 60°C pendant 15 heures, puis, après refroidissement à la température ambiante, est versé dans un mélange eau + glace. Le précipité jaune formé est filtré, ré-empaté dans l'eau puis séché sur P₂O₅. On obtient 2 g de 3-(4-nitro-2-methyl-phenylamino)-propane-1,2-diol (7)

### Etape 2 : synthèse du 3-(4-Amino-2-methyl-phenylamino)-propane-1,2-dioldichlorhydrate (8)

Le 3-(4-nitro-2-methyl-phenylamino)-propane-1,2-diol (7) précédemment obtenu est réduit avec un mélange zinc / chlorure d'ammonium/ Eau/ Ethanol bouillant. L'amine correspondante est isolée sous forme de dichlorhydrate.
Les spectres RMN du proton et de masse sont conformes à la structure attendue du produit.

### Exemple 5 : Synthèse du 3-(4-Amino-3-methyl-phenylamino)-propane-1,2-diol, dichlorhydrate (10)

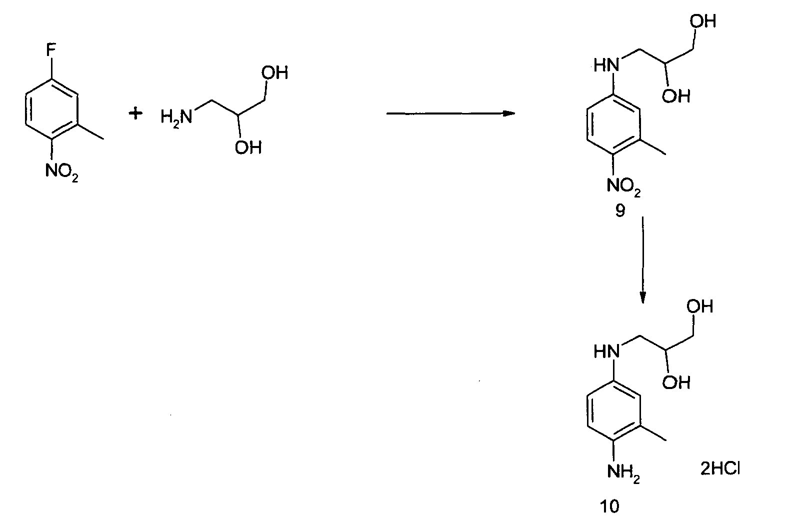

### Etape 1 : synthèse du 3-(4-nitro-3-methyl-phenylamino)-propane-1,2-diol (9)

A une solution de 20 ml de N-méthyl-pyrrolidinone, 1,41 g de 3-amino-1,2-propanediol et 1,57 g de triéthylamine, on ajoute 2 g de 5-fluoro-2-nitrotoluène. Le milieu réactionnel est chauffé à 60°C pendant 10 heures, puis, après refroidissement à la température ambiante, est versé dans un mélange eau + glace. Le milieu résultant est extrait à l'acétate d'éthyle puis la phase organique est concentrée sous vide. On obtient 0,28 g de 3-(4-nitro-3-methyl-phenylamino)-propane-1,2-diol (9).

### Etape 2 : synthèse du 3-(4-Amino-3-methyl-phenylamino)-propane-1,2-dioldichlorhydrate (10)

Le 3-(4-nitro-3-methyl-phenylamino)-propane-1,2-diol précédemment (9) obtenu est réduit avec un mélange zinc / chlorure d'ammonium/ Eau/ Ethanol bouillant. L'amine correspondante est isolée sous forme de dichlorhydrate.

Les spectres RMN du proton et de masse sont conformes à la structure attendue du produit.

### Exemple 6: Synthèse du 2-(4-Amino-3-methyl-phenylamino)-propane-1,3-diol, dichlorhydrate (12)

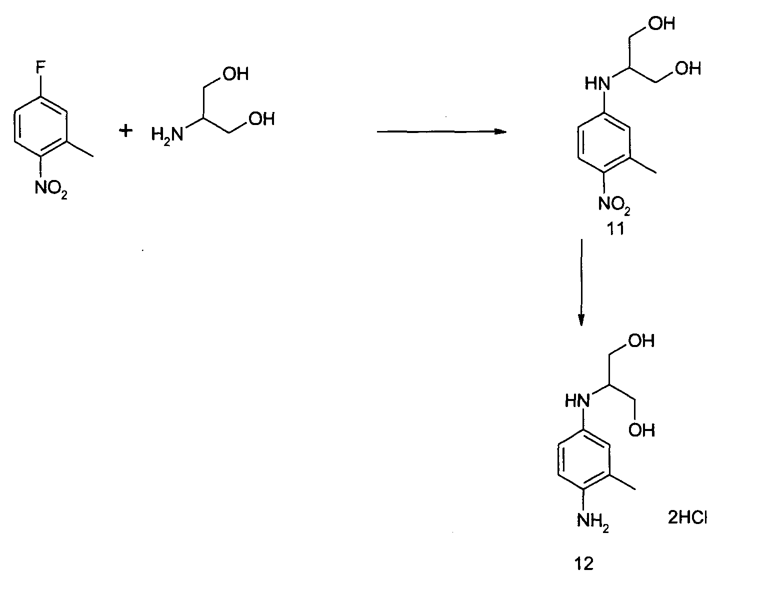

### Etape 1 : synthèse du 2-(4-nitro-3-methyl-phenylamino)-propane-1,3-diol (11)

A une solution de 20 ml de N-méthyl-pyrrolidinone, 1,4 g de 2-amino-1,3-propanediol et 1,57 g de triéthylamine, on ajoute 2 g de 5-fluoro-2-nitrotoluène. Le milieu réactionnel est chauffé à 60°C pendant 10 heures, puis, après refroidissement à la température ambiante, est versé dans un mélange eau + glace. Le milieu résultant est extrait à l'acétate d'éthyle puis la phase organique est concentrée sous vide. On obtient 1,45 g 2-(4-nitro-3-methyl-phenylamino)-propane-1,3-diol (11).

### Etape 2 : synthèse du 2-(4-Amino-3-methyl-phenylamino)-propane-1,3-diol, dichlorhydrate (12)

Le 2-(4-nitro-3-methyl-phenylamino)-propane-1,3-diol (11) précédemment obtenu est réduit avec un mélange zinc / chlorure d'ammonium/ Eau/ Ethanol bouillant. L'amine correspondante est isolée sous forme de dichlorhydrate.

Les spectres RMN du proton et de masse sont conformes à la structure attendue du produit.

### Exemple 7 : Synthèse du 2-(4-Amino-2-methyl-phenylamino)-propane-1,3-diol, dichlorhydrate (14)

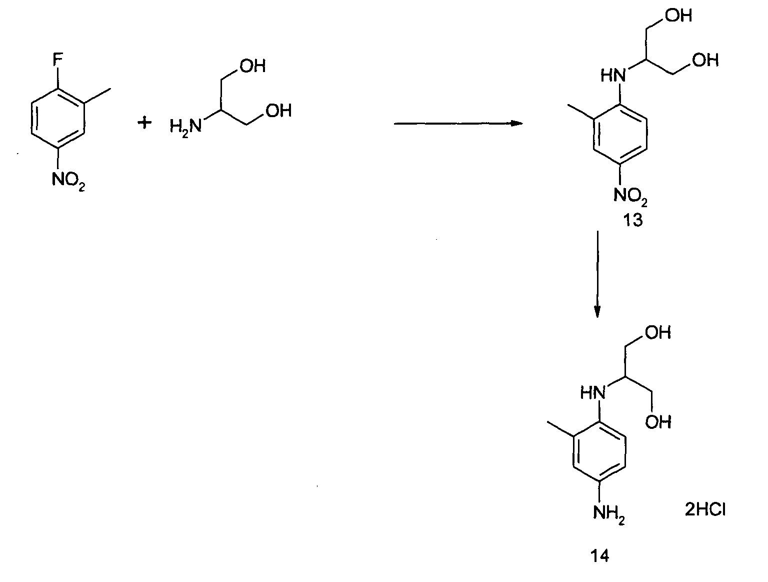

### Etape 1 : synthèse du 2-(4-nitro-2-methyl-phenylamino)-propane-1,3-diol (13)

A une solution de 20 ml de N-méthyl-pyrrolidinone, 1,4 g de 2-amino-1,3-propanediol et 1,57 g de triéthylamine, on ajoute 2 g de 2-fluoro-5-nitrotoluène. Le milieu réactionnel est chauffé à 60°C pendant 10 heures, puis, après refroidissement à la température ambiante, est versé dans un mélange eau + glace. Le milieu résultant est extrait à l'acétate d'éthyle puis la phase organique est concentrée sous vide. On obtient 0,32 g de 2-(4-nitro-2-methyl-phenylamino)-propane-1,3-diol (13).

### Etape 2 : synthèse du 2-(4-Amino-2-methyl-phenylamino)-propane-1,3-diol, dichlorhydrate (14)

Le 2-(4-nitro-2-methyl-phenylamino)-propane-1,3-diol (13) précédemment obtenu est réduit avec un mélange zinc / chlorure d'ammonium/ Eau/ Ethanol bouillant. L'amine correspondante est isolée sous forme de dichlorhydrate.

Les spectres RMN du proton et de masse sont conformes à la structure attendue du produit.

### Exemple 8 : Synthèse du 2-(4-Amino-phenylamino)-propane-1,3-diol, dichlorhydrate (16)

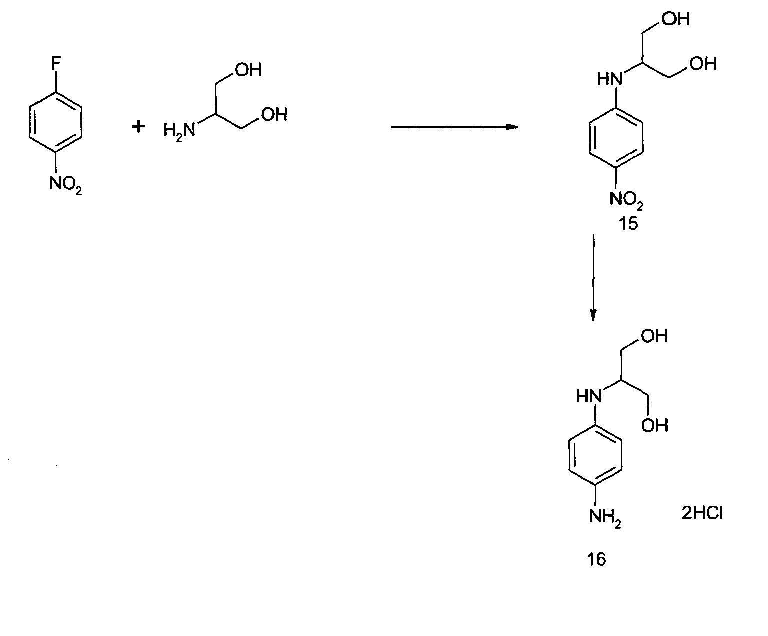

### Etape 1 : synthèse du 2-(4-nitro-phenylamino)-propane-1,3-diol (15).

A une solution de 20 ml de N-méthyl-pyrrolidinone, 1,55 g de 2-amino-1,3-propanediol et 2,35 g de K₂CO₃ , on ajoute 2 g de para-fluoro-nitrobenzène. Le milieu réactionnel est chauffé à 60°C pendant 15 heures, puis, après refroidissement à la température ambiante, est versé dans un mélange eau + glace. Le précipité jaune formé est filtré, ré-empaté dans l'eau puis séché sur P₂O₅. On obtient 0,1 g de 2-(4-nitro-phenylamino)-propane-1,3-diol (15) après purification sur colonne de silice.

### Etape 2 : synthèse du 2-(4-Amino-phenylamino)-propane-1,3-diol, dichlorhydrate (16)

Le 2-(4-nitro-phenylamino)-propane-1,3-diol (15) précédemment obtenu est réduit avec un mélange zinc / chlorure d'ammonium/ Eau/ Ethanol bouillant. L'amine correspondante est isolée sous forme de dichlorhydrate.

Les spectres RMN du proton et de masse sont conformes à la structure attendue du produit.

### Exemple 9 : Synthèse du 2-[[2-(4-Amino-2-methyl-phenylamino)-ethyl]-(2-hydroxy-ethyl)-amino]-ethanol, dichlorhydrate (18)

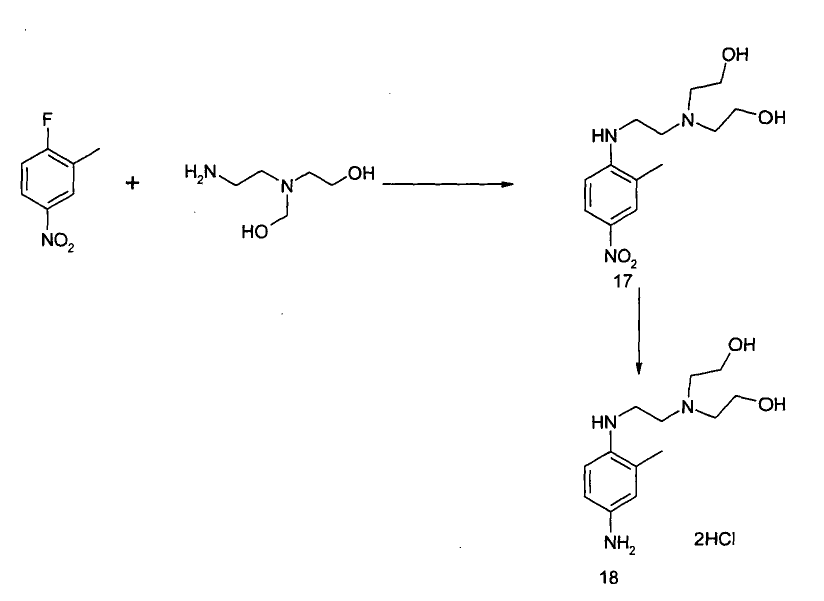

### Etape 1 : synthèse du 2-[[2-(4-nitro-2-methyl-phenylamino)-ethyl]-(2-hydroxyethyl)amino]ethanol (17)

A une solution de 30 ml de N-méthyl-pyrrolidinone, 2,29 g de N,N-bis(2-hydroxyéthyl)éthylènediamine et 1,72 g de triéthylamine, on ajoute 2 g de 2-fluoro-5-nitrotoluène. Le milieu réactionnel est chauffé à 80°C pendant 10 heures, puis, après refroidissement à la température ambiante, est versé dans un mélange eau + glace. Le milieu résultant est extrait à l'acétate d'éthyle puis la phase organique est concentrée sous vide. On obtient 1,5 g de 2-[[2-(4-nitro-2-methyl-phenylamino)-ethyl]-(2-hydroxyethyl)amino]ethanol (17)

### Etape 2 : synthèse du 2-[[2-(4-Amino-2-methyl-phenylamino)-ethyl]-(2-hydroxyethyl)-amino]-ethanol, dichlorhydrate (18)

Le 2-[[2-(4-nitro-2-methyl-phenylamino)-ethyl]-(2-hydroxyethyl)amino] ethanol (17) précédemment obtenu est réduit avec un mélange zinc / chlorure d'ammonium/ Eau/ Ethanol bouillant. L'amine correspondante est isolée sous forme de dichlorhydrate.

Les spectres RMN du proton et de masse sont conformes à la structure attendue du produit.

### Exemple 10 : Synthèse du 2-[[2-(4-Amino-phenylamino)-ethyl]-(2-hydroxyethyl)-amino]-ethanol, dichlorhydrate (20)

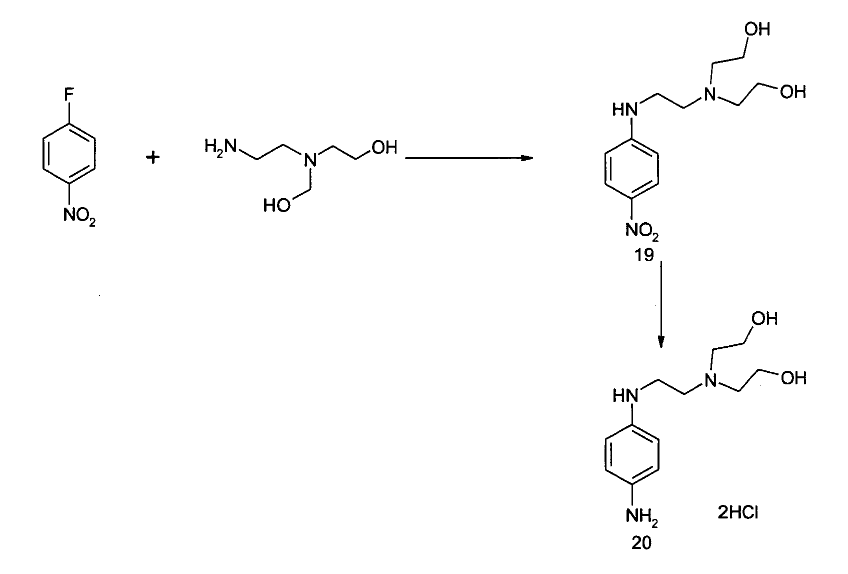

### Etape 1 : synthèse du 2-[[2-(4-nitroo-phenylamino)-ethyl]-(2-hydroxy-ethyl)-amino]-ethanol (19)

A une solution de 60 ml de N-méthyl-pyrrolidinone, 6,29 g de N,N-bis(2-hydroxyethyl)ethylenediamine et 4,3 g de triéthylamine , on ajoute 5 g de para-fluoro-nitrobenzène. Le milieu réactionnel est chauffé à 60°C pendant 15 heures, puis, après refroidissement à la température ambiante, est versé dans un mélange eau + glace. Le précipité jaune formé est filtré, ré-empaté dans l'eau puis séché sur P₂O₅. On obtient 7,5 g de 2-[[2-(4-nitroo-phenylamino)-ethyl]-(2-hydroxy-ethyl)-amino]-ethanol (19).

### Etape 2 : synthèse du 2-[[2-(4-Amino-phenylamino)-ethyl]-(2-hydroxy-ethyl)-amino]-ethanol, dichlorhydrate 20)

Le 2-[[2-(4-nitroo-phenylamino)-ethyl]-(2-hydroxy-ethyl)-amino]-ethanol (19) précédemment obtenu est réduit avec un mélange zinc / chlorure d'ammonium/ Eau/ Ethanol bouillant. L'amine correspondante est isolée sous forme de dichlorhydrate.

Les spectres RMN du proton et de masse sont conformes à la structure attendue du produit.

### Exemple 11 : Synthèse du 2-(4-Amino-2-methyl-phenylamino)-octadecane-1,3-diol (22

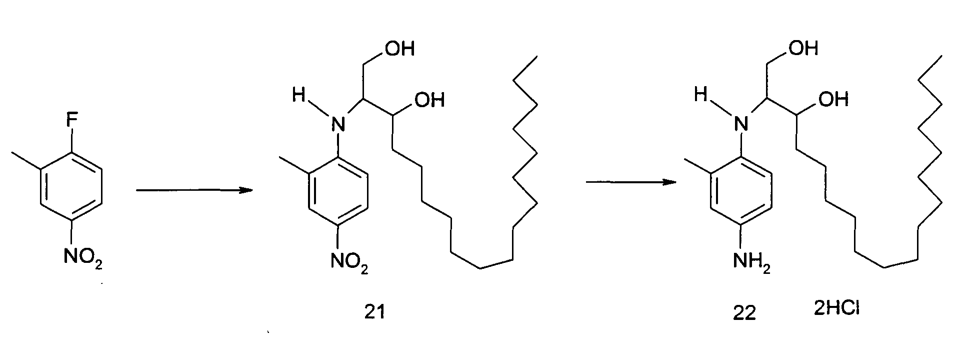

### Etape 1 : préparation du 2-[(2-methyl-4-nitrophenyl)amino]octadecane-1,3-diol (21)

Dans un tricol, on introduit sous azote 51 mg (0,00033 mole) de 2-fluoro-5-nitrotoluène, 37 mg (0,00035 mole) de carbonate de sodium et 3 ml de NMP. On ajoute goutte-à-goutte avec agitation 100 mg (0,00033 mole) de DL-1,3-dihydroxy-2-aminooctadecane (DL-dihydrosphingosine) dans 3 ml de NMP. On chauffe jusqu'au 90 °C. Après 6 jours de réaction, le mélange réactionnel est refroidit puis 5 ml d'eau distillée est ajouté lentement avec forte agitation. Un précipité semi-solide jaune foncé apparaît. Celui-ci est filtré, lavé plusieurs fois à l'eau, puis au pentane et séché sous vide. Le produit brut obtenu est purifié sur une colonne de silice, éluant : acétate d'éthyle / heptane : 2 / 3. On obtient 35 mg de dérivé nitré (21) attendu sous forme d'un solide jaune.

### Etape 2 : préparation du 2-(4-Amino-2-methyl-phenylamino)-octadecane-1,3-diol (22)

Le 2-[(2-methyl-4-nitrophenyl)amino]octadecane-1,3-diol (21)précédemment obtenu est réduit avec un mélange zinc / chlorure d'ammonium/ Eau/ Ethanol bouillant. L'amine correspondante est isolée sous forme de dichlorhydrate.
Les spectres RMN du proton et de masse sont conformes à la structure attendue du produit.

### -B- EXEMPLES DE TEINTURE

### Exemples 1 à 10 : Composition tinctoriale à partir du 2-(4-Amino-phénylamino)-octadécane-1,3-diol, dichlorhydrate (4)

### - Exemples 1 à 6 : Teinture en milieu acide

Les compositions tinctoriales suivantes sont préparées :

| **Exemple** | **1** | **2** | **3** | **4** | **5** | **6** |
|---|---|---|---|---|---|---|
| 2-(4-Amino-phenylamino)-octadecane-1,3-diol, dichlorhydrate (4) | 10-3 mole | 10-3 mole | 10-3 mole | 10-3 mole | 10-3 mole | 10-3 mole |
| Benzene-1,3-diol | 10-3 mole | | | | | |
| 5-Amino-2-methyl-phenol | | 10-3 mole | | | | |
| 1H-Indol-6-ol | | | 10-3 mole | | | |
| 2-Amino-pyridin-3-ol | | | | 10-3 mole | | |
| 3,6-Dimethyl-1H-pyrazolo [5,1-c][1,2,4]triazole | | | | | 10-3 mole | |
| 2-(2,4-Diamino-phenoxy)-ethanol,chlorhydrate | | | | | | 10-3 mole |
| Support de teinture (1) | (*) | (*) | (*) | (*) | (*) | (*) |
| Eau déminéralisée q.s.p. | 100g | 100g | 100g | 100g | 100g | 100g |

| | | | | | | |
|---|---|---|---|---|---|---|
| (*) : support de teinture (1) pH 7 | | | | | | |

| | |
|---|---|
| Alcool éthylique à 96° | 20,8 g |
| Méta-bisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en C₈-C₁₀ polyglucoside en solution aqueuse à 60% | 3,6 g M.A |
| Alcool benzylique | 2,0 g |
| Polyéthylène glycol à 8 motifs d'oxyde d'éthylène | 3,0 g |
| Na₂HPO₄ | 0,28 g |
| KH₂PO₄ | 0,46 g |

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pose, les mèches sont rinçées, lavées avec un shampooing standard, rinçées à nouveau puis séchées.

Les nuances obtenues figurent dans le tableau ci-dessous:

| **Exemple** | **1** | **2** | **3** | **4** | **5** | **6** |
|---|---|---|---|---|---|---|
| **Nuance observée** | gris intense | gris violet-bleu intense | brun rouge intense | gris intense | gris intense | violet-bleu intense |

### - Exemples 7 à 10 : teinture en milieu acide Basique

Les compositions tinctoriales suivantes sont préparées :

| **Exemple** | **7** | **8** | **9** | **10** |
|---|---|---|---|---|
| 2-(4-Amino-phenylamino)-octadecane-1,3-diol, dichlorhydrate (4) | 10-3 mole | 10-3 mole | 10-3 mole | 10-3 mole |
| 5-Amino-2-methyl-phenol | 10-3 mole | | | |
| 1H-Indol-6-ol | | 10-3 mole | | |
| 3,6-Dimethyl-1H-pyrazolo [5,1-c][1,2 ,4]triazole | | | 10-3 mole | |
| 2-(2,4-Diamino-phenoxy)-ethanol,chlorhydrate | | | | 10-3 mole |
| Support de teinture (2) | (*) | (*) | (*) | (*) |
| Eau déminéralisée q.s.p. | 100g | 100g | 100g | 100g |

| | | | | |
|---|---|---|---|---|
| (*) : support de teinture (2) pH 9.5 | | | | |

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en C₈-C₁₀ polyglucoside en solution aqueuse à 60% | 3,6 g M.A |
| Alcool benzylique | 2,0 g |
| Polyéthylène glycol à 8 motifs d'oxyde d'éthylène | 3,0 g |
| NH₄Cl | 4,32 g |
| Ammoniaque à 20% de NH₃ | 2,94 g |

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pose, les mèches sont rinçées, lavées avec un shampooing standard, rinçées à nouveau puis séchées.

Les nuances obtenues figurent dans le tableau ci-dessous :

| **Exemple** | **7** | **8** | **9** | **10** |
|---|---|---|---|---|
| **Nuance observée** | rouge | orangé | rouge chromatique | bleu intense |

### Exemples 11 à 17 : Composition tinctoriale à partir du 2-méthylpropane-1,3-diol, dichlorhydrate (2)

### - Exemples 11 à 14 : Teinture en milieu acide

Les compositions tinctoriales suivantes sont préparées :

| **Exemple** | **11** | **12** | **13** | **14** |
|---|---|---|---|---|
| 2-methyl-propane-1,3-diol, dichlorhydrate (2) | 10-3 mole | 10-3 mole | 10-3 mole | 10-3 mole |
| 1H-Indol-6-ol | 10-3 mole | | | |
| 2-Amino-pyridin-3-ol | | 10-3 mole | | |
| 2-(2,4-Diamino-phenoxy)-ethanol,chlorhydrate | | | 10-3 mole | |
| 3-Amino-2-chloro-6-methyl-phenol,chlorhydrate | | | | 10-3 mole |
| Support de teinture (1) | (*) | (*) | (*) | (*) |
| Eau déminéralisée q.s.p. | 100g | 100g | 100g | 100g |

| | | | | |
|---|---|---|---|---|
| (*) : support de teinture (1) pH 7 | | | | |

| | |
|---|---|
| Alcool éthylique à 96° | 20,8 g |
| Méta-bisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en C8-C10 polyglucoside en solution aqueuse à60% | 3,6 g M.A |
| Alcool benzylique | 2,0 g |
| Polyéthylène glycol à 8 motifs d'oxyde d'éthylène | 3,0 g |
| Na₂HPO₄ | 0,28 g |
| KH₂PO₄ | 0,46 g |

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pose, les mèches sont rinçées, lavées avec un shampooing standard, rinçées à nouveau puis séchées.

Les nuances obtenues figurent dans le tableau ci-dessous :

| **Exemple** | **11** | **12** | **13** | **14** |
|---|---|---|---|---|
| **Nuance observée** | brun orangé | brun rouge | gris bleu | gris violet |

### - Exemples 15 à 17 : teinture en milieu basique

Les compositions tinctoriales suivantes sont préparées :

| **Exemple** | **15** | **16** | **17** |
|---|---|---|---|
| 2-methyl-propane-1,3-diol, dichlorhydrate (2) | 10-3 mole | 10-3 mole | 10-3 mole |
| 5-Amino-2-methyl-phenol | 10-3 mole | | |
| 2-(2,4-Diamino-phenoxy)-ethanol,chlorhydrate | | 10-3 mole | |
| 3-Amino-2-chloro-6-methyl-phenol,chlorhydrate | | | 10-3 mole |
| Support de teinture (2) | (*) | (*) | (*) |
| Eau déminéralisée q.s.p. | 100g | 100g | 100g |

| | | | |
|---|---|---|---|
| (*) : support de teinture (2) pH 9.5 | | | |

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en C₈-C₁₀ polyglucoside en solution aqueuse à 60% | 3,6 g M.A |
| Alcool benzylique | 2,0 g |
| Polyéthylène glycol à 8 motifs d'oxyde d'éthylène | 3,0 g |
| NH₄Cl | 4,32 g |
| Ammoniaque à 20% de NH₃ | 2,94 g |

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pose, les mèches sont rinçées, lavées avec un shampooing standard, rinçées à nouveau puis séchées.

Les nuances obtenues figurent dans le tableau ci-dessous :

| **Exemple** | **15** | **16** | **17** |
|---|---|---|---|
| **Nuance observée** | rouge | bleu | violet |

### Exemples 18 à 23 : Composition tinctoriale à partir du 2-(4-Amino-phénylamino)-2-hydroxyméthyl-propane-1,3-diol(6)

### - Exemples 18 à 21 : Teinture en milieu acide

Les compositions tinctoriales suivantes sont préparées :

| **Exemple** | **18** | **19** | **20** | **21** |
|---|---|---|---|---|
| 2-(4-Amino-phenylamino)-2-hydroxymethyl-propane-1,3-diol(6) | 10-3 mole | 10-3 mole | 10-3 mole | 10-3 mole |
| 1H-Indol-6-ol | 10-3 mole | | | |
| 2-Amino-pyridin-3-ol | | 10-3 mole | | |
| 2-(2,4-Diamino-phenoxy)-ethanol,chlorhydrate | | | 10-3 mole | |
| 3-Amino-2-chloro-6-methyl-phenol,chlorhydrate | | | | 10-3 mole |
| Support de teinture (1) | (*) | (*) | (*) | (*) |
| Eau déminéralisée q.s.p. | 100g | 100g | 100g | 100g |

| | | | | |
|---|---|---|---|---|
| (*) : support de teinture (1) pH 7 | | | | |

| | |
|---|---|
| Alcool éthylique à 96° | 20,8 g |
| Méta-bisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en C8-C10 polyglucoside en solution aqueuse à 60% | 3,6 g M.A |
| Alcool benzylique | 2,0 g |
| Polyéthylène glycol à 8 motifs d'oxyde d'éthylène | 3,0 g |
| Na₂HPO₄ | 0,28 g |
| KH₂PO₄ | 0,46 g |

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pose, les mèches sont rinçées, lavées avec un shampooing standard, rinçées à nouveau puis séchées.

Les nuances obtenues figurent dans le tableau ci-dessous :

| **Exemple** | **18** | **19** | **20** | **21** |
|---|---|---|---|---|
| **Nuance observée** | brun orangé | brun rouge | bleu | violet |

### - Exemples 22 à 23 : teinture en milieu basique

Les compositions tinctoriales suivantes sont préparées :

| **Exemple** | **22** | **23** |
|---|---|---|
| 2-(4-Amino-phenylamino)-2-hydroxymethyl-propane-1,3-diol(6) | 10-3 mole | 10-3 mole |
| 2-(2,4-Diamino-phenoxy)-ethanol,chlorhydrate | 10-3 mole | |
| 3-Amino-2-chloro-6-methyl-phenol,chlorhydrate | | 10-3 mole |
| Support de teinture (2) | (*) | (*) |
| Eau déminéralisée q.s.p. | 100g | 100g |

| | | |
|---|---|---|
| (*) : support de teinture (2) pH 9,5 | | |

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en C₈-C₁₀ polyglucoside en solution aqueuse à 60% | 3,6 g M.A |
| Alcool benzylique | 2,0 g |
| Polyéthylène glycol à 8 motifs d'oxyde d'éthylène | 3,0 g |
| NH₄Cl | 4,32 g |
| Ammoniaque à 20% de NH₃ | 2,94 g |

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pose, les mèches sont rinçées, lavées avec un shampooing standard, rinçées à nouveau puis séchées.

Les nuances obtenues figurent dans le tableau ci-dessous :

| **Exemple** | **bleu** | **23** |
|---|---|---|
| **Nuance observée** | **22** | violet |

### Exemples 24 à 32 : Composition tinctoriale à partir du 2-(4-Amino-phenylamino)-propane-1,3-diol, dichlorhydrate (16)

### - Exemples 24 à 29 : Teinture en milieu acide

Les compositions tinctoriales suivantes sont préparées :

| Exemple | 24 | 25 | 26 | 27 | 28 | 29 |
|---|---|---|---|---|---|---|
| 2-(4-Amino-phenylamino)-propane-1,3-diol, dichlorhydrate (16) | 10-3 mole | 10-3 mole | 10-3 mole | 10-3 mole | 10-3 mole | 10-3 mole |
| Benzene-1,3-diol | 10-3 mole | | | | | |
| 5-Amino-2-methyl-phenol | | 10-3 mole | | | | |
| 1H-Indol-6-ol | | | 10-3 mole | | | |
| 3,6-Dimethyl-1H-pyrazolo[5,1-c][1,2 ,4]triazole | | | | 10-3 mole | | |
| 2-(2,4-Diamino-phenoxy)-ethanol,chlorhydra te | | | | | 10-3 mole | |
| 3-Amino-2-chloro-6-methyl-phenol,chlorhydra te | | | | | | 10-3 mole |
| Support de teinture (2) | (*) | (*) | (*) | (*) | (*) | (*) |
| Eau déminéralisée q.s.p. | 100g | 100g | 100g | 100g | 100g | 100g |

| | | | | | | |
|---|---|---|---|---|---|---|
| (*) : support de teinture (1) pH 7 | | | | | | |

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en C8-C10 polyglucoside en solution aqueuse à 60% | 3,6 g M.A |
| Alcool benzylique | 2,0 g |
| Polyéthylène glycol à 8 motifs d'oxyde d'éthylène | 3,0 g |
| Na₂HPO₄ | 0,28 g |
| KH₂PO₄ | 0,46 g |

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| Exemple | 24 | 25 | 26 | 27 | 28 | 29 |
|---|---|---|---|---|---|---|
| Nuance observée | brun jaune | violet-rouge intense | brun | brun rouge | gris bleu intense | violet-bleu intense |

### - Exemples 30 à 32 : teinture en milieu basique

Les compositions tinctoriales suivantes sont préparées :

| Exemple | 30 | 31 | 32 |
|---|---|---|---|
| 2-(4-Amino-phenylamino)-propane-1,3-diol, dichlorhydrate (16) | 10-3 mole | 10-3 mole | 10-3 mole |
| 5-Amino-2-methyl-phenol | 10-3 mole | | |
| 2-(2,4-Diamino-phenoxy)-ethanol,chlorhydrate | | 10-3 mole | |
| 3-Amino-2-chloro-6-methyl-phenol,chlorhydrate | | | 10-3 mole |
| Support de teinture (1) | (*) | (*) | (*) |
| Eau déminéralisée q.s.p. | 100g | 100g | 100g |

| | | | |
|---|---|---|---|
| (*) : support de teinture (2) pH 9.5 | | | |

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en C₈-C₁₀ polyglucoside en solution aqueuse à 60% | 3,6 g M.A |
| Alcool benzylique | 2,0 g |
| Polyéthylène glycol à 8 motifs d'oxyde d'éthylène | 3,0 g |
| NH₄Cl | 4,32 g |
| Ammoniaque à 20% de NH₃ | 2,94 g |

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| Exemple | 30 | 31 | 32 |
|---|---|---|---|
| Nuance observée | gris violet-rouge | bleu | violet-bleu intense |

### Exemples 33 à 42 : Composition tinctoriale à partir du 3-(4-Amino-2-methyl-phenylamino)-propane-1,2-diol, dichlorhydrate (8)

### - Exemples 33 à 38 : Teinture en milieu acide

Les compositions tinctoriales suivantes sont préparées :

| Exemple | 33 | 34 | 35 | 36 | 37 | 38 |
|---|---|---|---|---|---|---|
| 3-(4-Amino-2-methyl-phenylammo)-propane-1,2-diol, dichlorhydrate (8) | 10-3 mole | 10-3 mole | 10-3 mole | 10-3 mole | 10-3 mole | 10-3 mole |
| 5-Amino-2-methyl-phenol | 10-3 mole | | | | | |
| 1H-Indol-6-ol | | 10-3 mole | | | | |
| 2-Amino-pyridin-3-ol | | | 10-3 mole | | | |
| 3,6-Dimethyl-1H-pyrazolo[5,1-c][1,2 ,4]triazole | | | | 10-3 mole | | |
| 2-(2,4-Diamino-phenoxy)-ethanol,chlorhydrate | | | | | 10-3 mole | |
| 3-Amino-2-chloro-6-methyl-phenol,chlorhydrate | | | | | | 10-3 mole |
| Support de teinture (2) | (*) | (*) | (*) | (*) | (*) | (*) |
| Eau déminéralisée q.s.p. | 100g | 100g | 100g | 100g | 100g | 100g |

| | | | | | | |
|---|---|---|---|---|---|---|
| (*) : support de teinture (1) pH 7 | | | | | | |

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en C8-C10 polyglucoside en solution aqueuse à 60% | 3,6 g M.A |
| Alcool benzylique | 2,0 g |
| Polyéthylène glycol à 8 motifs d'oxyde d'éthylène | 3,0 g |
| Na₂HPO₄ | 0,28 g |
| KH₂PO₄ | 0,46 g |

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| Exemple | 33 | 34 | 35 | 36 | 37 | 38 |
|---|---|---|---|---|---|---|
| **Nuance observée** | **violet intense** | **brun** | **brun rouge intense** | **orangé** | **bleu intense** | **violet- bleu intense** |

### - Exemples 39 à 42 : teinture en milieu basique

Les compositions tinctoriales suivantes sont préparées :

| Exemple | 39 | 40 | 41 | 42 |
|---|---|---|---|---|
| 3-(4-Amino-2-methyl-phenylamino)-propane-1,2-diol, dichlorhydrate (8) | 10-3 mole | 10-3 mole | 10-3 mole | 10-3 mole |
| 5-Amino-2-methyl-phenol | 10-3 mole | | | |
| 3,6-Dimethyl-1H-pyrazolo[5,1-c][1,2 ,4]triazole | | 10-3 mole | | |
| 2-(2,4-Diamino-phenoxy)-ethanol,chlorhydrate | | | 10-3 mole | |
| 3-Amino-2-chloro-6-methyl-phenol,chlorhydrate | | | | 10-3 mole |
| Support de teinture (1) | (*) | (*) | (*) | (*) |
| Eau déminéralisée q.s.p. | 100g | 100g | 100g | 100g |

| | | | | |
|---|---|---|---|---|
| (*) : support de teinture (2) pH 9.5 | | | | |

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en C₈-C₁₀ polyglucoside en solution aqueuse à 60% | 3,6 g M.A |
| Alcool benzylique | 2,0 g |
| Polyéthylène glycol à 8 motifs d'oxyde d'éthylène | 3,0 g |
| NH₄Cl | 4,32 g |
| Ammoniaque à 20% de NH₃ | 2,94 g |

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| Exemple | 39 | 40 | 41 | 42 |
|---|---|---|---|---|
| Nuance observée | violet-rouge | rouge chromatique | bleu intense | violet-bleu intense |

### Exemples 43 à 51 : Composition tinctoriale à partir du 3-(4-Amino-3-methyl-phenylamino)-propane-1,2-diol, dichlorhydrate (10)

### - Exemples 43 à 47 : Teinture en milieu acide

Les compositions tinctoriales suivantes sont préparées :

| Exemple | 43 | 44 | 45 | 46 | 47 |
|---|---|---|---|---|---|
| 3-(4-Amino-3-methyl-phenylamino)-propane-1,2-diol, dichlorhydrate (10) | 10-3 mole | 10-3 mole | 10-3 mole | 10-3 mole | 10-3 mole |
| 5-Amino-2-methyl-phenol | 10-3 mole | | | | |
| 1H-Indol-6-ol | | 10-3 mole | | | |
| 2-Amino-pyridin-3-ol | | | 10-3 mole | | |
| 2-(2,4-Diamino-phenoxy)-ethanol,chlorhydrate | | | | 10-3 mole | |
| 3-Amino-2-chloro-6-methyl-phenol,chlorhydrate | | | | | 10-3 mole |
| Support de teinture (2) | (*) | (*) | (*) | (*) | (*) |
| Eau déminéralisée q.s.p. | 100g | 100g | 100g | 100g | 100g |

| | | | | | |
|---|---|---|---|---|---|
| (*) : support de teinture (1) pH 7 | | | | | |

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en C8-C10 polyglucoside en solution aqueuse à 60% | 3,6 g M.A |
| Alcool benzylique | 2,0 g |
| Polyéthylène glycol à 8 motifs d'oxyde d'éthylène | 3,0 g |
| Na₂HPO₄ | 0,28 g |
| KH₂PO₄ | 0,46 g |

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pose, les mèches sont rinçées, lavées avec un shampooing standard, rinçées à nouveau puis séchées.

Les nuances obtenues figurent dans le tableau ci-dessous :

| Exemple | 43 | 44 | 45 | 46 | 47 |
|---|---|---|---|---|---|
| Nuance observée | gris violet intense | brun orangé | brun jaune | gris vert-bleu intense | bleu intense |

### - Exemples 48 à 51 : teinture en milieu basique

Les compositions tinctoriales suivantes sont préparées :

| Exemple | 48 | 49 | 50 | 51 |
|---|---|---|---|---|
| 3-(4-Amino-3-methyl-phenylamino)-propane-1,2-diol, dichlorhydrate (10) | 10-3 mole | 10-3 mole | 10-3 mole | 10-3 mole |
| 5-Amino-2-methyl-phenol | 10-3 mole | | | |
| 3,6-Dimethyl-1H-pyrazolo[5,1-c][1,2 ,4]triazole | | 10-3 mole | | |
| 2-(2,4-Diamino-phenoxy)-ethanol,chlorhydrate | | | 10-3 mole | |
| 3-Amino-2-chloro-6-methyl-phenol,chlorhydrate | | | | 10-3 mole |
| Support de teinture (1) | (*) | (*) | (*) | (*) |
| Eau déminéralisée q.s.p. | 100g | 100g | 100g | 100g |

| | | | | |
|---|---|---|---|---|
| (*) : support de teinture (2) pH 9.5 | | | | |

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en C₈-C₁₀ polyglucoside en solution aqueuse à 60% | 3,6 g M.A |
| Alcool benzylique | 2,0 g |
| Polyéthylène glycol à 8 motifs d'oxyde d'éthylène | 3,0 g |
| NH₄Cl | 4,32 g |
| Ammoniaque à 20% de NH₃ | 2,94 g |

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| Exemple | 48 | 49 | 50 | 51 |
|---|---|---|---|---|
| Nuance observée | violet | rouge chromatiqu e | vert-bleu | bleu intense |

### Exemples 52 à 60 : Composition tinctoriale à partir du 2-(4-Amino-3-methyl-phenylamino)-propane-1,3-diol, dichlorhydrate (12)

### - Exemples 52 à 56: Teinture en milieu acide

Les compositions tinctoriales suivantes sont préparées :

| Exemple | 52 | 53 | 54 | 55 | 56 |
|---|---|---|---|---|---|
| 2-(4-Amino-3-methyl-phenylamino)-propane-1,3-diol, dichlorhydrate (12) | 10-3 mole | 10-3 mole | 10-3 mole | 10-3 mole | 10-3 mole |
| 5-Amino-2-methyl-phenol | 10-3 mole | | | | |
| 1H-Indol-6-ol | | 10-3 mole | | | |
| 2-Amino-pyridin-3-ol | | | 10-3 mole | | |
| 2-(2,4-Diamino-phenoxy)-ethanol,chlorhydrate | | | | 10-3 mole | |
| 3-Amino-2-chloro-6-methyl-phenol,chlorhydrate | | | | | 10-3 mole |
| Support de teinture (2) | (*) | (*) | (*) | (*) | (*) |
| Eau déminéralisée q.s.p. | 100g | 100g | 100g | 100g | 100g |

| | | | | | |
|---|---|---|---|---|---|
| (*) : support de teinture (1) pH 7 | | | | | |

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en C8-C10 polyglucoside en solution aqueuse à 60% | 3,6 g M.A |
| Alcool benzylique | 2,0 g |
| Polyéthylène glycol à 8 motifs d'oxyde d'éthylène | 3,0 g |
| Na₂HPO₄ | 0,28 g |
| KH₂PO₄ | 0,46 g |

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pose, les mèches sont rinçées, lavées avec un shampooing standard, rinçées à nouveau puis séchées.

Les nuances obtenues figurent dans le tableau ci-dessous :

| Exemple | 52 | 53 | 54 | 55 | 56 |
|---|---|---|---|---|---|
| Nuance observée | gris violet intense | brun rouge | brun orangé | gris vert-bleu intense | bleu intense |

### - Exemples 57 à 60 : teinture en milieu basique

Les compositions tinctoriales suivantes sont préparées :

| Exemple | 57 | 58 | 59 | 60 |
|---|---|---|---|---|
| 2-(4-Amino-3-methyl-phenylamino)-propane-1,3-diol, dichlorhydrate (12) | 10-3 mole | 10-3 mole | 10-3 mole | 10-3 mole |
| 5-Amino-2-methyl-phenol | 10-3 mole | | | |
| 3,6-Dimethyl-1H-pyrazolo[5,1-c][1,2 ,4]triazole | | 10-3 mole | | |
| 2-(2,4-Diamino-phenoxy)-ethanol,chlorhydrate | | | 10-3 mole | |
| 3-Amino-2-chloro-6-methyl-phenol,chlorhydrate | | | | 10-3 mole |
| Support de teinture (1) | (*) | (*) | (*) | (*) |
| Eau déminéralisée q.s.p. | 100g | 100g | 100g | 100g |

| | | | | |
|---|---|---|---|---|
| (*) : support de teinture (2) pH 9.5 | | | | |

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en C₈-C₁₀ polyglucoside en solution aqueuse à 60% | 3,6 g M.A |
| Alcool benzylique | 2,0 g |
| Polyéthylène glycol à 8 motifs d'oxyde d'éthylène | 3,0 g |
| NH₄Cl | 4,32 g |
| Ammoniaque à 20% de NH₃ | 2,94 g |

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| Exemple | 57 | 58 | 59 | 60 |
|---|---|---|---|---|
| Nuance observée | gris violet | rouge chromatique | vert-bleu | bleu intense |

### Exemples 61 à 74 : Composition tinctoriale à partir du 2-[[2-(4-Amino-phenylamino)-ethyl]-(2-hydroxy-ethyl)-amino]-ethanol, dichlorhydrate (20)

### - Exemples 61 à 67 : Teinture en milieu acide

Les compositions tinctoriales suivantes sont préparées :

| Exemple | 61 | 62 | 63 | 64 | 65 | 66 | 67 |
|---|---|---|---|---|---|---|---|
| 2-[[2-(4-Amino-phenylamino)-ethyl]-(2-hydroxy-ethyl)-amino]-ethanol, dichlorhydrate (20) | 10-3 mole | 10-3 mole | 10-3 mole | 10-3 mole | 10-3 mole | 10-3 mole | 10-3 mole |
| Benzene-1,3-diol | 10-3 mole | | | | | | |
| 5-Amino-2-methyl-phenol | | 10-3 mole | | | | | |
| 1H-Indol-6-ol | | | 10-3 mole | | | | |
| 2-Amino-pyridin-3-ol | | | | 10-3 mole | | | |
| 3,6-Dimethyl-1H-pyrazolo[5,1-c][1,2 ,4]triazole | | | | | 10-3 mole | | |
| 2-(2,4-Diamino-phenoxy)-ethanol,chlorhydrate | | | | | | 10-3 mole | |
| 3-Amino-2-chloro-6-methyl-phenol,chlorhydrate | | | | | | | 10-3 mole |
| Support de teinture (2) | (*) | (*) | (*) | (*) | (*) | (*) | (*) |
| Eau déminéralisée q.s.p. | 100g | 100g | 100g | 100g | 100g | 100g | 100g |

| | | | | | | | |
|---|---|---|---|---|---|---|---|
| (*) : support de teinture (1) pH 7 | | | | | | | |

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en C8-C10 polyglucoside en solution aqueuse à 60% | 3,6 g M.A |
| Alcool benzylique | 2,0 g |
| Polyéthylène glycol à 8 motifs d'oxyde d'éthylène | 3,0 g |
| Na₂HPO₄ | 0,28 g |
| KH₂PO₄ | 0,46 g |

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pose, les mèches sont rinçées, lavées avec un shampooing standard, rinçées à nouveau puis séchées.

Les nuances obtenues figurent dans le tableau ci-dessous :

| Exemple | 61 | 62 | 63 | 64 | 65 | 66 | 67 |
|---|---|---|---|---|---|---|---|
| Nuance observée | brun orangé intense | rouge intense | brun rouge | rouge intense | orangé | gris bleu intense | violet-rouge intense |

### - Exemples 68 à 74 : teinture en milieu basique

Les compositions tinctoriales suivantes sont préparées :

| Exemple | 68 | 69 | 70 | 71 | 72 | 73 | 74 |
|---|---|---|---|---|---|---|---|
| 2-[[2-(4-Amino-phenylamino)-ethyl]-(2-hydroxy-ethyl)-amino]-ethanol, dichlorhydrate (20) | 10-3 mole | 10-3 mole | 10-3 mole | 10-3 mole | 10-3 mole | 10-3 mole | 10-3 mole |
| Benzene-1,3-diol | 10-3 mole | | | | | | |
| 5-Amino-2-methyl-phenol | | 10-3 mole | | | | | |
| 1H-Indol-6-ol | | | 10-3 mole | | | | |
| 2-Amino-pyridin-3-ol | | | | 10-3 mole | | | |
| 3,6-Dimethyl-1H-pyrazolo[5,1-c][1,2 ,4]triazole | | | | | 10-3 mole | | |
| 2-(2,4-Diamino-phenoxy)-ethanol,chlorhydrate | | | | | | 10-3 mole | |
| 3-Amino-2-chloro-6-methyl-phenol,chlorhydrate | | | | | | | 10-3 mole |
| Support de teinture (1) | (*) | (*) | (*) | (*) | (*) | (*) | (*) |
| Eau déminéralisée q.s.p. | 100g | 100g | 100g | 100g | 100g | 100g | 100g |

| | | | | | | | |
|---|---|---|---|---|---|---|---|
| (*) : support de teinture (2) pH 9.5 | | | | | | | |

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en C₈-C₁₀ polyglucoside en solution aqueuse à 60% | 3,6 g M.A |
| Alcool benzylique | 2,0 g |
| Polyéthylène glycol à 8 motifs d'oxyde d'éthylène | 3,0 g |
| NH₄Cl | 4,32 g |
| Ammoniaque à 20% de NH₃ | 2,94 g |

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| Exemple | 68 | 69 | 70 | 71 | 72 | 73 | 74 |
|---|---|---|---|---|---|---|---|
| Nuance observée | orange | rouge | orange | rouge | rouge chromatiq e | violet-bleu intense | violet intense |

### Exemples 75 à 82 : Composition tinctoriale à partir du 2-(4-Amino-2-methyl-phenylamino)-propane-1,3-diol, dichlorhydrate (14)

### - Exemples 75 à 79 : Teinture en milieu acide

Les compositions tinctoriales suivantes sont préparées :

| Exemple | 75 | 76 | 77 | 78 | 79 |
|---|---|---|---|---|---|
| 2-(4-Amino-2-methyl-phenylamino)-propane-1,3-diol, dichlorhydrate (14) | 10-3 mole | 10-3 mole | 10-3 mole | 10-3 mole | 10-3 mole |
| 5-Amino-2-methyl-phenol | 10-3 mole | | | | |
| 1H-Indol-6-ol | | 10-3 mole | | | |
| 2-Amino-pyridin-3-ol | | | 10-3 mole | | |
| 2-(2,4-Diamino-phenoxy)-ethanol,chlorhydrate | | | | 10-3 mole | |
| 3-Amino-2-chloro-6-methyl-phenol,chlorhydrate | | | | | 10-3 mole |
| Support de teinture (2) | (*) | (*) | (*) | (*) | (*) |
| Eau déminéralisée q.s.p. | 100g | 100g | 100g | 100g | 100g |

| | | | | | |
|---|---|---|---|---|---|
| (*) : support de teinture (1) pH 7 | | | | | |

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en C8-C10 polyglucoside en solution aqueuse à 60% | 3,6 g M.A |
| Alcool benzylique | 2,0 g |
| Polyéthylène glycol à 8 motifs d'oxyde d'éthylène | 3,0 g |
| Na₂HPO₄ | 0,28 g |
| KH₂PO₄ | 0,46 g |

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pose, les mèches sont rinçées, lavées avec un shampooing standard, rinçées à nouveau puis séchées.

Les nuances obtenues figurent dans le tableau ci-dessous :

| Exemple | 75 | 76 | 77 | 78 | 79 |
|---|---|---|---|---|---|
| Nuance observée | violet intense | brun | brun | bleu intense | violet-bleu intense |

### - Exemples 80 à 82 : teinture en milieu basique

Les compositions tinctoriales suivantes sont préparées :

| Exemple | 81 | 82 |
|---|---|---|
| 2-(4-Amino-2-methyl-phenylamino)-propane-1,3-diol, dichlorhydrate (14) | 10-3 mole | 10-3 mole |
| 2-(2,4-Diamino-phenoxy)-ethanol,chlorhydrate | 10-3 mole | |
| 3-Amino-2-chloro-6-methyl-phenol,chlorhydrate | | 10-3 mole |
| Support de teinture (1) | (*) | (*) |
| Eau déminéralisée q.s.p. | 100g | 100g |

| | | |
|---|---|---|
| (*) : support de teinture (2) pH 9.5 | | |

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en C₈-C₁₀ polyglucoside en solution aqueuse à 60% | 3,6 g M.A |
| Alcool benzylique | 2,0 g |
| Polyéthylène glycol à 8 motifs d'oxyde d'éthylène | 3,0 g |
| NH₄Cl | 4,32 g |
| Ammoniaque à 20% de NH₃ | 2,94 g |

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| Exemple | 81 | 82 |
|---|---|---|
| Nuance observée | bleu | violet-bleu intense |

### Exemples 83 à 93 : Composition tinctoriale à partir du 2-[[2-(4-Amino-2-methyl-phenylamino)-ethyl]-(2-hydroxy-ethyl)-amino]-ethanol, dichlorhydrate (18)

### - Exemples 83 à 89 : Teinture en milieu acide

Les compositions tinctoriales suivantes sont préparées :

| Exemple | 83 | 84 | 85 | 86 | 87 | 88 | 89 |
|---|---|---|---|---|---|---|---|
| 2-[[2-(4-Amino-2-methyl-phenylamino)-ethyl]-(2-hydroxy-ethyl)-amino]-ethanol, dichlorhydrate (18) | 10-3 mole | 10-3 mole | 10-3 mole | 10-3 mole | 10-3 mole | 10-3 mole | 10-3 mole |
| Benzene-1,3-diol | 10-3 mole | | | | | | |
| 5-Amino-2-methyl-phenol | | 10-3 mole | | | | | |
| 1H-Indol-6-ol | | | 10-3 mole | | | | |
| 2-Amino-pyridin-3-ol | | | | 10-3 mole | | | |
| 3,6-Dimethyl-1H-pyrazolo[5,1-c][1,2 ,4]triazole | | | | | 10-3 mole | | |
| 2-(2,4-Diamino-phenoxy)-ethanol,chlorhydrate | | | | | | 10-3 mole | |
| 3-Amino-2-chloro-6-methyl-phenol,chlorhydrate | | | | | | | 10-3 mole |
| Support de teinture (2) | (*) | (*) | (*) | (*) | (*) | (*) | (*) |
| Eau déminéralisée q.s.p. | 100g | 100g | 100g | 100g | 100g | 100g | 100g |

| | | | | | | | |
|---|---|---|---|---|---|---|---|
| (*) : support de teinture (1) pH 7 | | | | | | | |

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en C8-C10 polyglucoside en solution aqueuse à 60% | 3,6 g M.A |
| Alcool benzylique | 2,0 g |
| Polyéthylène glycol à 8 motifs d'oxyde d'éthylène | 3,0 g |
| Na₂HPO₄ | 0,28 g |
| KH₂PO₄ | 0,46 g |

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pose, les mèches sont rinçées, lavées avec un shampooing standard, rinçées à nouveau puis séchées.

Les nuances obtenues figurent dans le tableau ci-dessous :

| Exemple | 83 | 84 | 85 | 86 | 87 | 88 | 89 |
|---|---|---|---|---|---|---|---|
| Nuance observée | orangé | rouge intense | brun orangé | rouge intense | orangé | gris bleu intense | violet intense |

### - Exemples 90 à 93 : teinture en milieu basique

Les compositions tinctoriales suivantes sont préparées :

| Exemple | 90 | 91 | 92 | 93 |
|---|---|---|---|---|
| 2-[[2-(4-Amino-2-methyl-phenylamino)-ethyl]-(2-hydroxy-ethyl)-amino]-ethanol, dichlorhydrate (18) | 10-3 mole | 10-3 mole | 10-3 mole | 10-3 mole |
| 5-Amino-2-methyl-phenol | 10-3 mole | | | |
| 3,6-Dimethyl-1H-pyrazolo[5,1-c][1,2 ,4]triazole | | 10-3 mole | | |
| 2-(2,4-Diamino-phenoxy)-ethanol,chlorhydrate | | | 10-3 mole | |
| 3-Amino-2-chloro-6-methyl-phenol,chlorhydrate | | | | 10-3 mole |
| Support de teinture (1) | (*) | (*) | (*) | (*) |
| Eau déminéralisée q.s.p. | 100g | 100g | 100g | 100g |

| | | | | |
|---|---|---|---|---|
| (*) : support de teinture (2) pH 9.5 | | | | |

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en C₈-C₁₀ polyglucoside en solution aqueuse à 60% | 3,6 g M.A |
| Alcool benzylique | 2,0 g |
| Polyéthylène glycol à 8 motifs d'oxyde d'éthylène | 3,0 g |
| NH₄Cl | 4,32 g |
| Ammoniaque à 20% de NH₃ | 2,94 g |

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| Exemple | 90 | 91 | 92 | 93 |
|---|---|---|---|---|
| Nuance observée | rouge | rouge chromatique | bleu intense | violet intense |

## Revendications

1. Composé **caractérisé en ce qu'**il s'agit d'une para-phénylènediamine secondaire de formule générale (I): 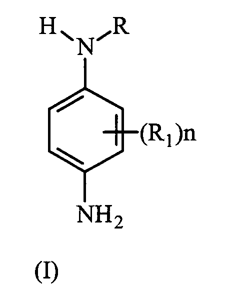 dans laquelle :
R représente un radical alkyle en C₂-C₁₈ linéaire ou ramifié, non substitué ou substitué par un ou plusieurs groupements amino, mono ou dialkyl(C₁-C₁₅)amino, alkyl(C₁-C₁₅)carbonyle, amido, mono ou dialkyl(C₁-C₁₅)amino carbonyle, ce radical alkyle pouvant être remplacépar un ou plusieurs hétéroatomes choisis parmi l'oxygène ou l'azote,
le radical R comprenant de 2 à 4 groupements hydroxyles ;
R₁ représente un atome d'hydrogène, un radical alkyle en C₁-C₁₅, un alcoxy en C₁-C₁₅, un hydroxy(alcoxy en (C₁-C₁₅)), un (alcoxy en (C₁-C₁₅))alkyle en C₁-C₁₅, un mono ou poly-hydroxy alkyle en C₁-C₁₅, ou un atome d'halogène;
n représente un entier variant de 1 à 4 ;
ou ses sels d'addition
à l'exception de la N(2,3-dihydroxypropyl) para-phénylènediamine.

2. Composé selon la revendication 1, **caractérisé en ce que** le groupement R de la formule (I) représente un radical alkyle en C₂-C₁₈ linéaire ou ramifié comportant au moins 2 à 4 groupements hydroxyles, ce radical alkyle pouvant être interrompu par un ou plusieurs hétéroatomes choisis parmi l'oxygène et l'azote.

3. Composé selon la revendication 1 ou 2, **caractérisé en ce que** le groupement R₁ de la formule (I) représente un atome d'hydrogène, un radical alkyle en C₁-C₆ ou un alcoxy en C₁-C₆.

4. Composé selon l'une quelconque des revendications 1 à 3, **caractérisé en ce que** le composé de formule (I) est choisi parmi les composés suivants:
-2-[[2-(4-Amino-phénylamino)-éthyl]-(2-hydroxy-éthyl)-amino]-éthanol ;
-2-(4-Amino-phénylamino)-2-hydroxyméthyl-propane-1,3-diol ;
-2-(4-Amino-phénylamino)-octadécane-1,3-diol ;
-2-(4-Amino-phénylamino)-2-méthyl-propane-1,3-diol ;
-2-(4-Amino-phénylamino)-propane-1,3-diol ;
-2-(4-Amino-phénylamino)-2-éthyl-propane-1,3-diol ;
-2-(4-Amino-phénylamino)-2-hydroxyméthyl-propane-1,3-diol ;
-3-(4-Amino-2-méthyl-phénylamino)-propane-1,2-diol ;
-2-(4-Amino-2-méthyl-phénylamino)-2-hydroxyméthyl-propane-1,3-diol ;
-2-(4-Amino-2-méthyl-phénylamino)-octadécane-1,3-diol;
-2-(4-Amino-2-méthyl-phénylamino)-2-méthyl-propane-1,3-diol;
-2-(4-Amino-2-méthyl-phénylamino)-propane-1,3-diol;
-2-(4-Amino-2-méthyl-phénylamino)-2-éthyl-propane-1,3-diol;
-2-(4-Amino-2-méthyl-phénylamino)-2-hydroxyméthyl-propane-1,3-diol;
-2-[[2-(4-Amino-2-méthyl-phénylamino)-éthyl]-(2-hydroxy-éthyl)-amino]-éthanol;
-3-(4-Amino-3-méthyl-phénylamino)-propane-1,2-diol;
-2-(4-Amino-3-méthyl-phénylamino)-2-hydroxyméthyl-propane-1,3-diol;
-2-(4-Amino-3-méthyl-phénylamino)-octadécane-1,3-diol;
-2-(4-Amino-3-méthyl-phénylamino)-2-méthyl-propane-1,3-diol;
-2-(4-Amino-3-méthyl-phénylamino)-propane-1,3-diol;
-2-(4-Amino-3-méthyl-phénylamino)-2-éthyl-propane-1,3-diol;
-2-(4-Amino-3-méthyl-phénylamino)-2-hydroxyméthyl-propane-1,3-diol;
-2-[[2-(4-Amino-3-méthyl-phénylamino)-éthyl]-(2-hydroxy-éthyl)-amino]-éthanol;
-3-[3-[4-Amino-phénylamino)-propoxyl-propane-1,2-diol;
-2-[[4-(4-Amino-phénylamino)-butyl]-(2-hydroxy-éthyl)-amino]-éthanol.

5. Composé selon l'une quelconque des revendications 1 à 4, **caractérisé en ce que** les sels d'addition avec un acide des para-phénylènediamines secondaires de formule générale (I) sont choisis parmi les chlorhydrates, les bromhydrates, les sulfates, les citrates, les succinates, les tartrates, les lactates, les tosylates, les benzènesulfonates, les phosphates et les acétates.

6. Composé nitré **caractérisé en ce qu'**il possède la structure suivante (II) : 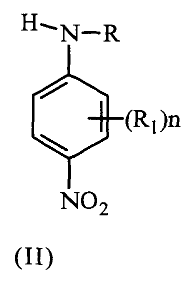 dans laquelle :
R représente un radical alkyle en C₂-C₁₈ linéaire ou ramifié, non substitué ou substitué par un ou plusieurs groupements amino, mono ou dialkyl(C₁-C₁₅)amino, alkyl(C₁-C₁₅)carbonyle, amido, mono ou dialkyl(C₁-C₁₅)amino carbonyle, un ou plusieurs atomes de carbone de ce radical alkyle pouvant être remplacépar un ou plusieurs hétéroatomes choisis parmi l'oxygène ou l'azote,
Le radical R comprenant de 2 à 4 groupements hydroxyles ;
R₁ représente un atome d'hydrogène, un radical alkyle en C₁-C₁₅, un alcoxy en C₁-C₁₅, un hydroxy(alcoxy en (C₁-C₁₅)), un (alcoxy en (C₁-C₁₅))alkyle en C₁-C₁₅, un mono ou poly-hydroxy alkyle en C₁-C₁₅, ou un atome d'halogène;
n représente un entier variant de 1 à 4.

7. Procédé de préparation du composé de formule (I) tel que défini dans les revendications 1 à 5, **caractérisé par le fait qu'**on effectue une étape de réduction d'un composé nitré de formule (II) tel que défini à la revendication 6.

8. Utilisation du composé de formule (I) suivante : 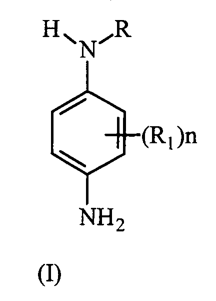 dans laquelle :
R représente un radical alkyle en C₂-C₁₈ linéaire ou ramifié, non substitué ou substitué par un ou plusieurs groupements amino, mono ou dialkyl(C₁-C₁₅)amino, alkyl(C₁-C₁₅)carbonyle, amido, mono ou dialkyl(C₁-C₁₅)amino carbonyle, ce radical alkyle pouvant être interrompu par un ou plusieurs hétéroatomes choisis parmi l'oxygène ou l'azote,
Le radical R comprenant de 2 à 4 groupements hydroxyles ;
R₁ représente un atome d'hydrogène, un radical alkyle en C₁-C₁₅, un alcoxy en C₁-C₁₅, un hydroxy(alcoxy en (C₁-C₁₅)), un (alcoxy en (C₁-C₁₅))alkyle en C₁-C₁₅, un mono ou poly-hydroxy alkyle en C₁-C₁₅, ou un atome d'halogène;
n représente un entier variant de 1 à 4 ;
à l'exception de la N(2,3-dihydroxypropyl) para-phénylènediamine ;
ou ses sels d'addition, pour la coloration d'oxydation capillaire.

9. Composition cosmétique pour la teinture des fibres kératiniques, de préférence les fibres kératiniques humaines, telles que les cheveux, comprenant, dans un milieu approprié pour la teinture, au moins un composé de formule générale (I) : 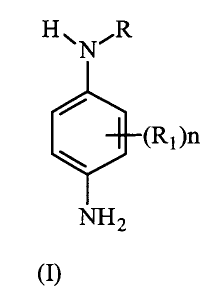 dans laquelle :
R représente un radical alkyle en C₂-C₁₈ linéaire ou ramifié, non substitué ou substitué par un ou plusieurs groupements amino, mono ou dialkyl(C₁-C₁₅)amino, alkyl(C₁-C₁₅)carbonyle, amido, mono ou dialkyl(C₁-C₁₅)amino carbonyle, ce radical alkyle pouvant être interrompu par un ou plusieurs hétéroatomes choisis parmi l'oxygène ou l'azote,
Le radical R comprenant de 2 à 4 groupements hydroxyles ;
R₁ représente un atome d'hydrogène, un radical alkyle en C₁-C₁₅, un alcoxy en C₁-C₁₅, un hydroxy(alcoxy en (C₁-C₁₅)), un (alcoxy en (C₁-C₁₅))alkyle en C₁-C₁₅, un mono ou poly-hydroxy alkyle en C₁-C₁₅, ou un atome d'halogène;
n représente un entier variant de 1 à 4 ;
à l'exception de la N(2,3-dihydroxypropyl) para-phénylènediamine ;
ou ses sels d'addition.

10. Composition selon la revendication 9, **caractérisé en ce que** le groupement R de la formule (I) représente un radical alkyle en C₂-C₁₈ linéaire ou ramifié comportant au moins 2 à 4 groupements hydroxyles, ce radical alkyle pouvant être interrompu par un ou plusieurs hétéroatomes choisis parmi l'oxygène et l'azote.

11. Composé selon la revendication 9 ou 10, **caractérisé en ce que** le groupement R₁ de la formule (I) représente un atome d'hydrogène, un radical alkyle en C₁-C₆ ou un alcoxy en C₁-C₆.

12. Composé selon l'une quelconque des revendications 9 à 11, **caractérisé en ce que** le composé de formule (I) est choisi parmi les composés suivants:
-[[2-(4-Amino-phénylamino)-éthyl]-(2-hydroxy-éthyl)-amino]-éthanol ;
-2-(4-Amino-phénylamino)-2-hydroxyméthyl-propane-1,3-diol ;
-2-(4-Amino-phénylamino)-octadécane-1,3-diol ;
-2-(4-Amino-phénylamino)-2-méthyl-propane-1,3-diol ;
-2-(4-Amino-phénylamino)-propane-1,3-diol ;
-2-(4-Amino-phénylamino)-2-éthyl-propane-1,3-diol ;
-2-(4-Amino-phénylamino)-2-hydroxyméthyl-propane-1,3-diol ;
-3-(4-Amino-2-méthyl-phénylamino)-propane-1,2-diol ;
-2-(4-Amino-2-méthyl-phénylamino)-2-hydroxyméthyl-propane-1,3-diol ;
-2-(4-Amino-2-méthyl-phénylamino)-octadécane-1,3-diol;
-2-(4-Amino-2-méthyl-phénylamino)-2-méthyl-propane-1,3-diol;
-2-(4-Amino-2-méthyl-phénylamino)-propane-1,3-diol;
-2-(4-Amino-2-méthyl-phénylamino)-2-éthyl-propane-1,3-diol;
-2-(4-Amino-2-méthyl-phénylamino)-2-hydroxyméthyl-propane-1,3-diol;
-2-[[2-(4-Amino-2-méthyl-phénylamino)-éthyl]-(2-hydroxy-éthyl)-amino]-éthanol;
-3-(4-Amino-3-méthyl-phénylamino)-propane-1,2-diol;
-2-(4-Amino-3-méthyl-phénylamino)-2-hydroxyméthyl-propane-1,3-diol;
-2-(4-Amino-3-méthyl-phénylamino)-octadécane-1,3-diol;
-2-(4-Amino-3-méthyl-phénylamino)-2-méthyl-propane-1,3-diol;
-2-(4-Amino-3-méthyl-phénylamino)-propane-1,3-diol;
-2-(4-Amino-3-méthyl-phénylamino)-2-éthyl-propane-1,3-diol;
-2-(4-Amino-3-méthyl-phénylamino)-2-hydroxyméthyl-propane-1,3-diol;
-2-[[2-(4-Amino-3-méthyl-phénylamino)-éthyl]-(2-hydroxy-éthyl)-amino]-éthanol;
-3-[3-[4-Amino-phénylamino)-propoxyl-propane-1,2-diol;
- 2-[[4-(4-Amino-phénylamino)-butyl]-(2-hydroxy-éthyl)-amino]-éthanol.

13. Composé selon l'une quelconque des revendications 9 à 12, **caractérisé en ce que** les sels d'addition avec un acide des para-phénylènediamines secondaires de formule générale (I) sont choisis parmi les chlorhydrates, les bromhydrates, les sulfates, les citrates, les succinates, les tartrates, les lactates, les tosylates, les benzènesulfonates, les phosphates et les acétates.

14. Composition selon l'une quelconque des revendications 9 à 13, **caractérisée en ce que** la concentration du composé de formule générale (I) est comprise entre 0,0001 et 20%, de préférence entre 0,005 à 6% en poids par rapport au poids total de la composition.

15. Composition selon l'une quelconque des revendications 9 à 14, **caractérisée en ce que** le milieu approprié pour la teinture est constitué par de l'eau ou par un mélange d'eau et d'au moins un solvant organique choisi parmi les alcools inférieurs en C₁-C₄, ramifiés ou non; les polyols et éthers de polyols; les alcools aromatiques et leurs mélanges.

16. Composition selon l'une quelconque des revendications 9 à 15, **caractérisée en ce qu'**elle comprend au moins un adjuvant cosmétique choisi parmi les agents anti-oxydants, les agents de pénétration, les agents séquestrants, les parfums, les tampons, les agents dispersants, les tensioactifs, les agents conditionneurs, les agents filmogènes, les polymères, les céramides, les agents conservateurs, les agents nacrants ou opacifiants, les vitamines ou provitamines.

17. Composition selon la revendication 16 **caractérisée en ce que** la quantité en adjuvant cosmétique est comprise pour chacun d'eux entre 0,01 et 20 % en poids par rapport au poids total de la composition.

18. Composition selon l'une quelconque des revendications 9 à 17, **caractérisée en ce que** la composition comprend au moins un coupleur d'oxydation choisi parmi les méta-phénylènediamines, les méta-aminophénols, les méta-diphénols, les coupleurs naphtaléniques, les coupleurs hétérocycliques ainsi que leurs sels d'addition.

19. Composition selon la revendication 18, **caractérisée en ce que** la concentration du ou des coupleurs est comprise entre 0,0001 et 20 % en poids par rapport au poids total de la composition.

20. Composition selon l'une quelconque des revendications 9 à 19, **caractérisée en ce que** la composition comprend au moins une base d'oxydation additionnelle différente du composé de formule (I) choisie parmi les para-phénylènediamines, les bis-phénylalkylènediamines, les para-aminophénols, les ortho-aminophénols, les bases hétérocycliques et leurs sels d'addition.

21. Composition selon la revendication 20, **caractérisée en ce que** la concentration de la ou des bases d'oxydation additionnelle est comprise entre 0,0001 et 20% en poids par rapport au poids total de la composition.

22. Composition selon l'une quelconque des revendications 9 à 21, **caractérisée en ce qu'**elle renferme un ou plusieurs colorants directs naturels ou cationiques.

23. Procédé de teinture des fibres kératiniques et en particulier des fibres kératiniques humaines telles que les cheveux, **caractérisée par le fait qu'**on applique sur lesdites fibres au moins une composition selon l'une quelconque des revendications 9 à 22, pendant une durée suffisante pour développer la coloration désirée en présence d'un agent oxydant.

24. Composition prête à l'emploi, **caractérisée en ce qu'**elle comprend dans une composition tinctoriale telle que définie dans l'une quelconque des revendications 9 à 22 au moins un agent oxydant choisi parmi le peroxyde d'hydrogène, le peroxyde d'urée, les bromates de métaux alcalins, les persels, les peracides et les enzymes oxydases, et de préférence le peroxyde d'hydrogène

25. Utilisation pour la teinture des fibres kératiniques et en particulier des fibres kératiniques humaines telles que les cheveux de la composition telle que définie dans l'une quelconque des revendications 9 à 22.

26. Dispositif à plusieurs compartiments dans lequel le premier compartiment contient une composition tinctoriale pour la teinture des fibres kératiniques telle que définie à l'une quelconque des revendications 9 à 22 et un deuxième compartiment contient un agent oxydant.
